# EUROPEAN PATENT APPLICATION

(11) **EP 3 187 153 A2**
(43) Date of publication of application: **05.07.2017**
(21) Application number: 16191617.6
(22) Date of filing: 29.09.2006
(51) Int. Cl.: A61F 2/38

(54) **BEARING IMPLANT**

(30) Priority: 30.09.2005 US 722171 P
(62) Divisional of application: 06815884.9
(71) Applicant: ConforMIS, Inc., Burlington, MA 01803 (US)
(72) Inventor: LANG, Philipp, Lexington, MA 02421 (US); BURDULIS,, Albert G. Jr., San Francisco, CA 94127 (US); FITZ, Wolfgang, Sherborn, MA 01770 (US); STEINES, Daniel, Palo Alto, CA 94303 (US)
(74) Representative: Grund, Martin

(57) **Abstract**

A mobile bearing implant includes a first component. The first component includes a bone facing surface for engaging one of a substantially uncut articular cartilage surface and a substantially uncut subchondral bone surface. The bone facing surface substantially matches the one of the articular cartilage surface and the subchondral bone surface. The mobile bearing implant further includes an external surface. A bearing component has a first surface for slidingly engaging the external surface of the first component, and a second surface for engaging at least one of a second component, bone, and cartilage.

## Description

### Technical Field

The present invention relates to orthopedic implants and systems, such as joint implants, interpositional implants, and mobile bearing implants.

### Background Art

A conventional prosthetic joint implant may include single or multiple components. For example, a joint implant often referred to as a mobile bearing implant may include a bearing component that is interposed between first and second components. The bearing component extends the range of movements that can be accommodated, such as sliding and rotational movement.

Implantation of these prosthetic devices is usually associated with loss of underlying tissue and bone and, with some devices, serious long-term complications associated with the loss of significant amount of tissue and bone can include infection, osteolysis and also loosening of the implant. Such joint arthroplasties can be highly invasive and require surgical resection of the entire, or a majority of the, articular surface of one or more bones involved in the repair. Typically with these procedures, the marrow space is fairly extensively reamed in order to fit the stem of the prosthesis within the bone. Reaming results in a loss of the patient's bone stock and over time subsequent osteolysis will frequently lead to loosening of the prosthesis. Further, the area where the implant and the bone mate degrades over time requiring the prosthesis to eventually be replaced. Since the patient's bone stock is limited, the number of possible replacement surgeries is also limited for joint arthroplasty. In short, over the course of 15 to 20 years, and in some cases even shorter time periods, the patient can run out of therapeutic options ultimately resulting in a painful, non-functional joint.

Another concern with prosthetic joint implants, such as a mobile bearing implant, is to ensure full range of appropriate motion. This must be balanced with the risk of dislocation of the device.

### Summary of the Invention

In accordance with a first embodiment of the invention, a mobile bearing implant includes a first component. The first component includes a bone facing surface for engaging one of a substantially uncut articular cartilage surface and a substantially uncut subchondral bone surface. The bone facing surface substantially matches the one of the articular cartilage surface and the subchondral bone surface. The mobile bearing implant further includes an external surface. A bearing component has a first surface for slidingly engaging the external surface of the first component, and a second surface for engaging at least one of a second component, bone, and cartilage.

In accordance with related embodiments of the invention, the bone facing surface of the first component may substantially match one of a substantially uncut articular cartilage surface of a tibia and a substantially uncut subchondral bone surface of a tibia, and the second surface of the bearing component engages a femoral implant component.

In further related embodiments of the invention, the external surface of the first component may be curved. The external surface may include a plurality of curved surfaces with varying radii. The external surface may be flat along at least one axis.

In yet further related embodiments of the invention, the external surface of the first component may include a slot, and the first surface of the bearing component includes an anchor. The anchor slidingly engages the slot so as to direct movement of the bearing component along the external surface of the first component. The slot may be curved. The slot may include a plurality of curvatures with varying radii. The slot may be sloped.

In still a further embodiment of the invention, the first component may include at least one stop for limiting motion of the bearing component, the stop including a curved surface for contacting the bearing component.

In accordance with another embodiment of the invention, a mobile bearing implant includes: a first component having a bone facing surface for engaging at least one of bone and cartilage; and an external surface. A bearing component has a first surface for slidingly engaging the external surface of the first component, and a second surface for engaging at least one of a second component, bone, and cartilage. The external surface includes at least one of a concavity and a convexity.

In accordance with related embodiments of the invention, the bone facing surface of the first component may engage a tibial articular surface, and the second surface of the bearing component engages a femoral implant component. The external surface of the first component may include a plurality of curved surfaces with varying radii. The external surface may be flat along at least one axis.

In accordance with further related embodiments of the invention, the external surface of the first component may includes a slot, and the first surface of the bearing component includes an anchor. The anchor slidingly engages the slot so as to direct movement of the bearing component along the external surface of the first component. The slot may be curved, or sloped. The slot may include a plurality of curvatures with varying radii.

In accordance with still further related embodiments of the invention, the first component may include at least one stop for limiting motion of the bearing component, the stop including a curved surface for contacting the bearing component.

In accordance with another embodiment of the invention, a mobile bearing implant includes a first component including: a bone facing surface for engaging one of bone and cartilage; and an external surface. The external surface includes a slot. A bearing component has a first surface for slidingly engaging the external surface of the first component, and a second surface for engaging at least one of a second component, bone, and cartilage. The first surface of the bearing component includes an anchor. The anchor slidingly engages the slot so as to direct movement of the bearing component along the external surface of the first component.

In accordance with related embodiments of the invention, the bone facing surface of the first component may engage a tibial articular surface, and the second surface of the bearing component engages a femoral implant component. The slot may be curved. The slot may include a plurality of curvatures with varying radii. The slot may be sloped.

In accordance with another embodiment of the invention, a mobile bearing implant includes: a first component having a bone facing surface for engaging one of bone and cartilage; and an external surface. A bearing component has a first surface and a second surface, the first surface for slidingly engaging the external surface of the first component, and the second surface for engaging at least one of a second component, bone, and cartilage. At least one of the first surface and the second surface includes a curved surface in one dimension, the curved surface having a plurality of radii.

In accordance with another embodiment of the invention, a mobile bearing implant includes: a first component having a bone facing surface for engaging one of bone and cartilage; and an external surface. A bearing component has a first surface and a second surface, the first surface for slidingly engaging the external surface of the first component, and the second surface for engaging at least one of a second component, bone, and cartilage. The first component has an outer perimeter of varying radii.

In accordance with related embodiments of the invention, the outer perimeter of the first component may be kidney shaped. The outer perimeter of the first component may be asymmetric. The first component may have a larger, or smaller, outer perimeter than the bearing component.

In accordance with embodiments related to the above-described embodiments, the first component may be fixedly anchored into an articular surface using one or more fins, keels, and/or pegs. The fins, keels and/or pegs may have various orientations and lengths. For example, the fins and/or pegs may be perpendicular to each other. The bone facing surface of the first component may sit on top of the natural surface of the subchondral bone or articular cartilage, with only the anchoring mechanism protruding into the bone.

In accordance with further embodiments related to the above-described embodiments, the first component may include one or more stops, that restrict motion of one or more components of the mobile bearing. The stop(s) may restrict motion in one or more dimensions. The stop(s) may be oriented in various directions. Each stop may include curved and/or straight portions For example, a stop may have a constant or variable radius.

The joint implants described herein may be implemented for the knee, hip, ankle, shoulder, elbow, wrist, and hand. The various joint implants described herein may be used, without limitation, in conjunction with knee implants, including a unicompartmental arthroplasty, medial or lateral; a bicompartmental arthroplasty that covers portions or all of one femoral condyle, medial or lateral, and the trochlea, and a total knee arthroplasty system. In a total knee arthroplasty system, the intercondylar region can be preserved by using a medial and a lateral tibial device in combination. Both devices may be a fixed, non-mobile bearing, both can be a mobile bearing, or one can be a fixed, non-mobile bearing, while the other is a mobile bearing.

### Brief Description of the Drawings

The foregoing features of the invention will be more readily understood by reference to the following detailed description, taken with reference to the accompanying drawings, in which:
**FIG. 1A** is a block diagram of a method for assessing a joint in need of repair according to the invention wherein the existing joint surface is unaltered, or substantially unaltered, prior to receiving the selected implant. **FIG. 1B** is a block diagram of a method for assessing a joint in need of repair according to the invention wherein the existing joint surface is unaltered, or substantially unaltered, prior to designing an implant suitable to achieve the repair. **FIG. 1C** is a block diagram of a method for developing an implant and using the implant in a patient.

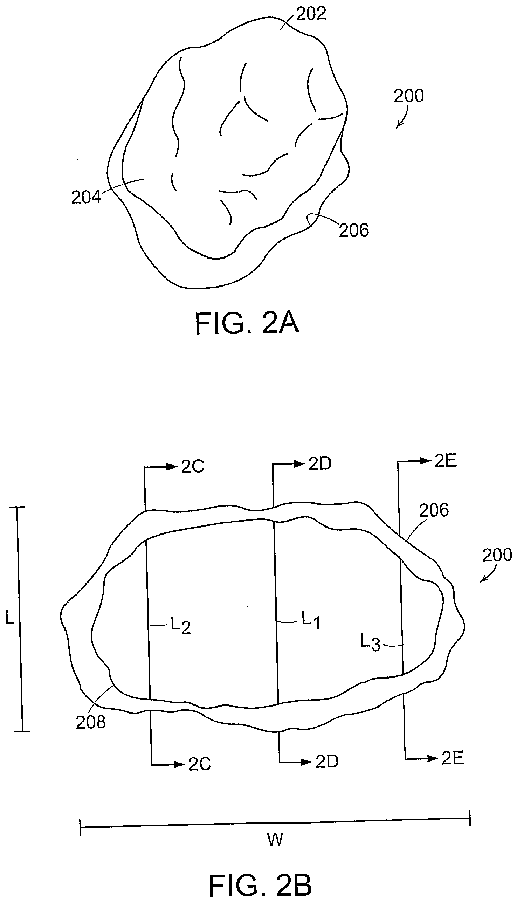

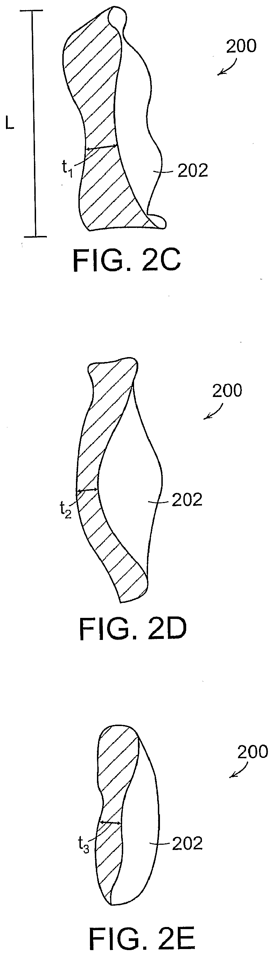

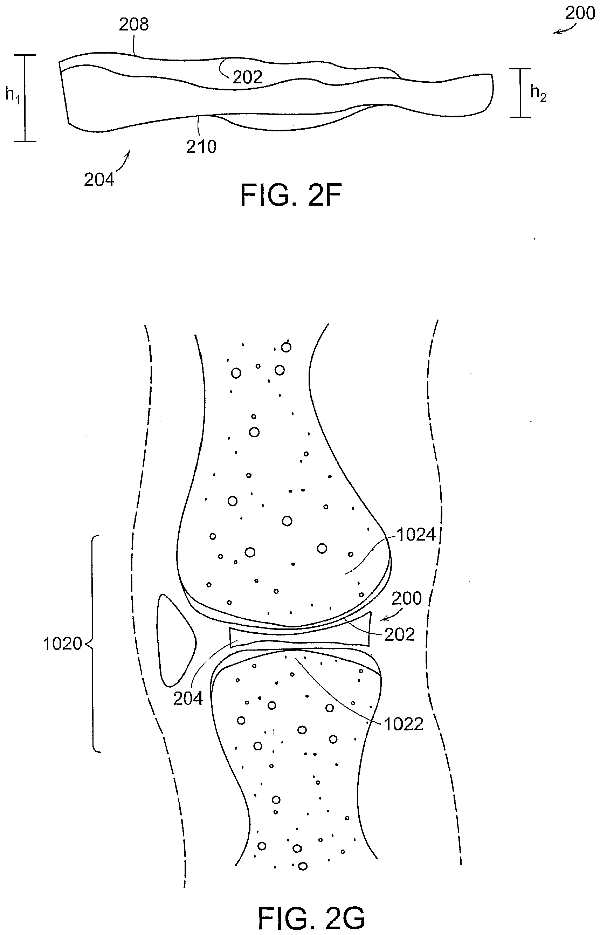

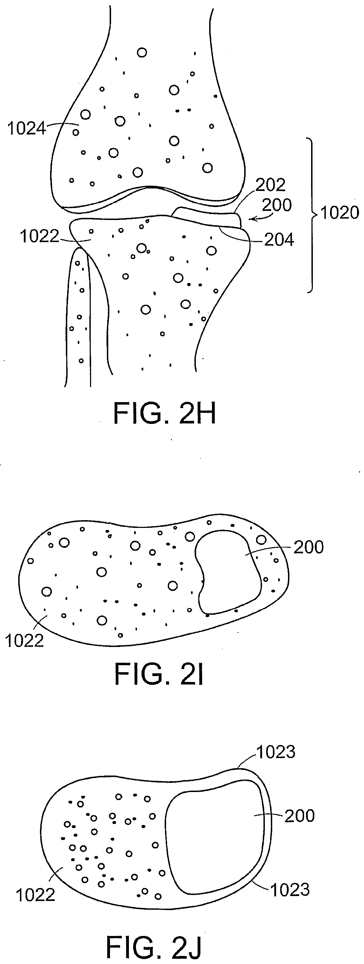

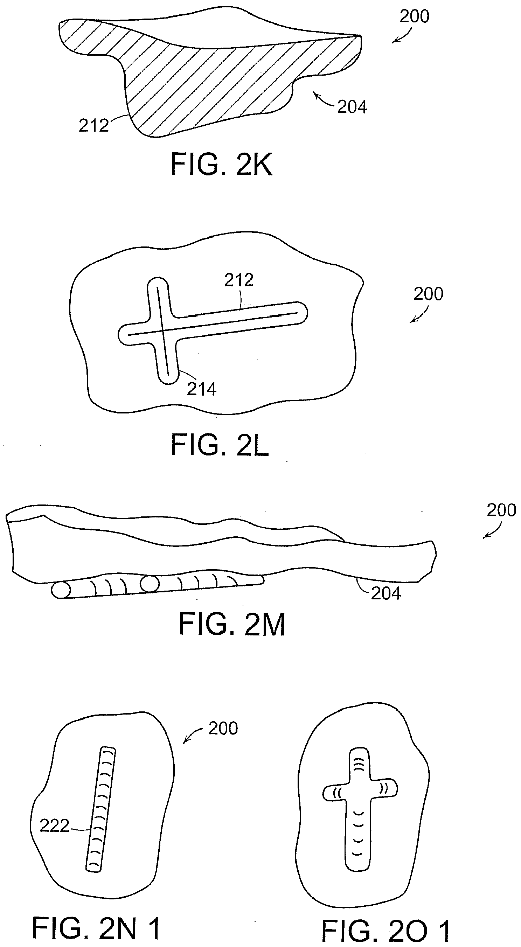

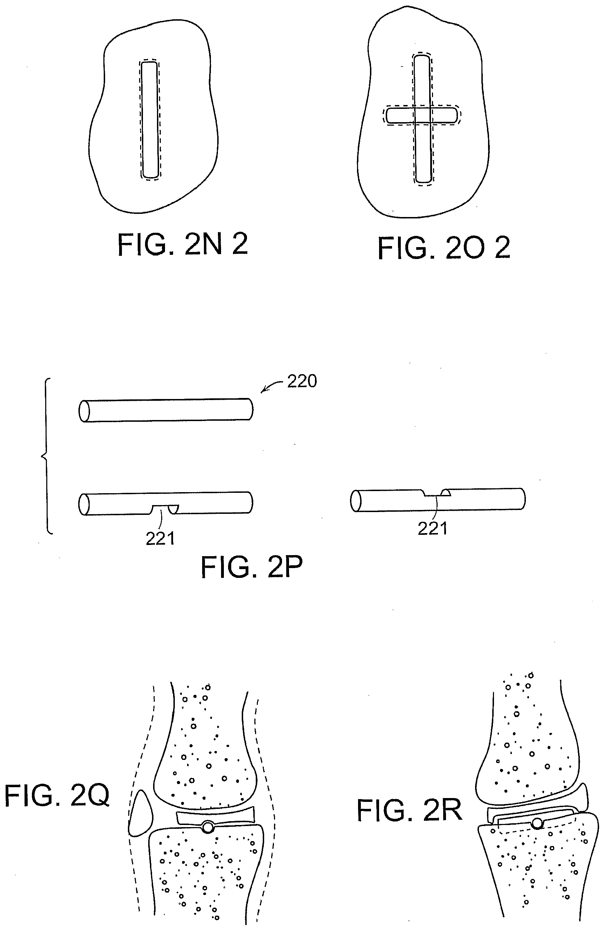

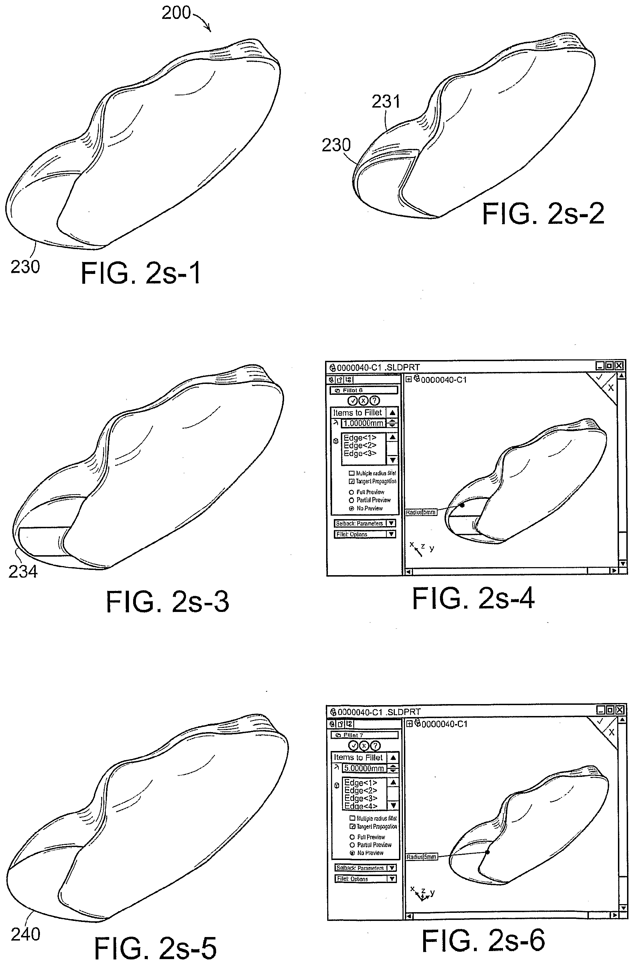

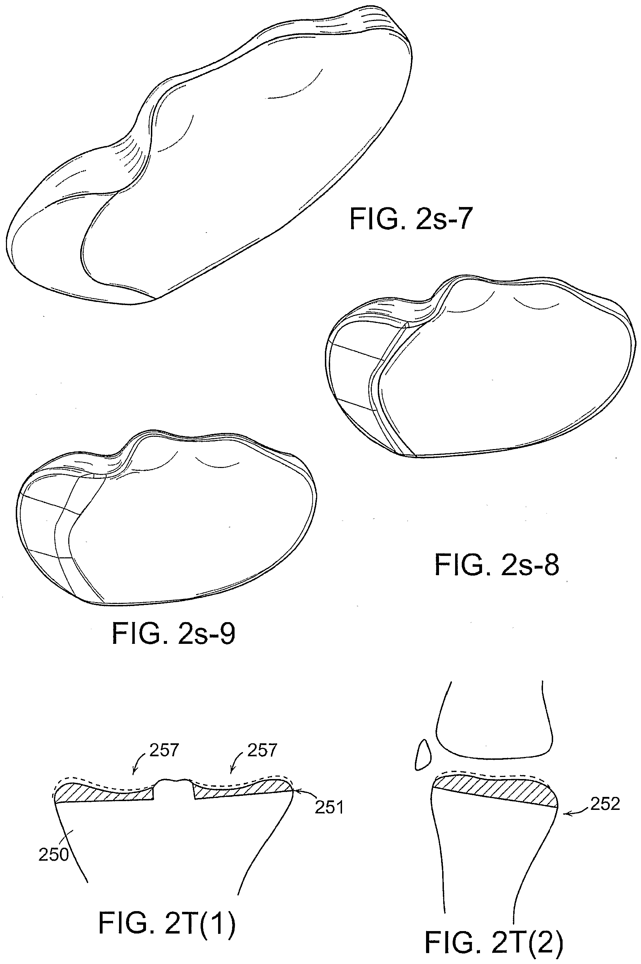

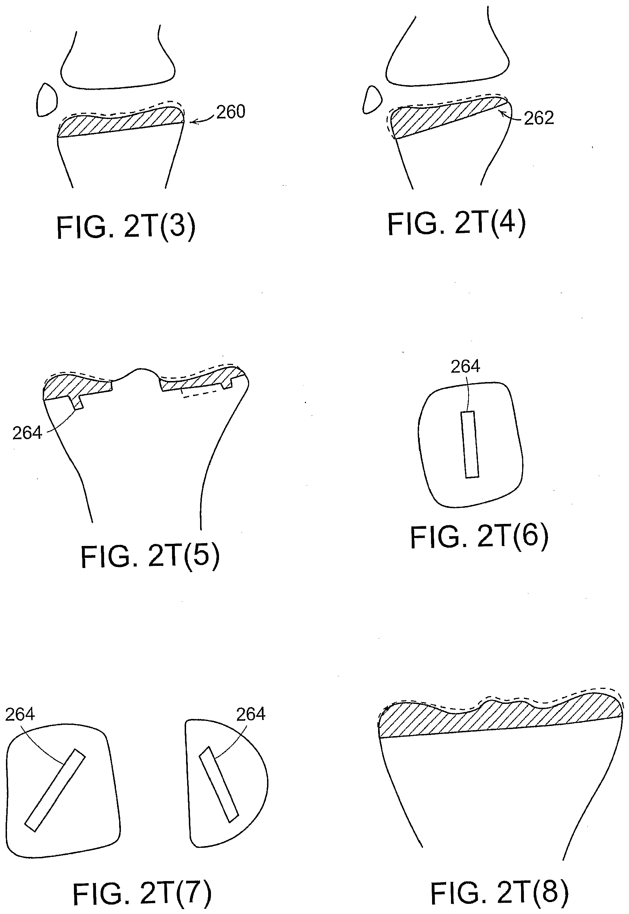

**FIG. 2A** is a perspective view of a joint implant of the invention suitable for implantation at the tibial plateau of the knee joint. **FIG. 2B** is a top view of the implant of **FIG. 2A****.** **FIG. 2C** is a cross-sectional view of the implant of **FIG. 2B** along the lines C-C shown in **FIG. 2B****.** **FIG. 2D** is a cross-sectional view along the lines D-D shown in **FIG. 2B****.** **FIG. 2E** is a cross-sectional view along the lines E-E shown in **FIG. 2B****.** **FIG. 2F** is a side view of the implant of **FIG. 2A****.** **FIG. 2G** is a cross-sectional view of the implant of **FIG. 2A** shown implanted taken along a plane parallel to the sagittal plane. **FIG. 2H** is a cross-sectional view of the implant of **FIG. 2A** shown implanted taken along a plane parallel to the coronal plane. **FIG. 2I** is a cross-sectional view of the implant of **FIG. 2A** shown implanted taken along a plane parallel to the axial plane. **FIG. 2J** shows a slightly larger implant that extends closer to the bone medially (towards the edge of the tibial plateau) and anteriorly and posteriorly. **FIG. 2K** is a side view of an alternate embodiment of the joint implant of **FIG. 2A** showing an anchor in the form of a keel. **FIG. 2L** is a bottom view of an alternate embodiment of the joint implant of **FIG. 2A** showing an anchor. **FIG. 2M** shows an anchor in the form of a cross-member. **FIG. 2N-O** are alternative embodiments of the implant showing the lower surface have a trough for receiving a cross-bar. **FIG. 2P** illustrates a variety of cross-bars. **FIGS. 2Q-R** illustrate the device implanted within a knee joint. **FIGS. 2S(1-9)** illustrate another implant suitable for the tibial plateau further having a chamfer cut along one edge. **FIG. 2T(1-8)**illustrate an alternate embodiment of the tibial implant wherein the surface of the joint is altered to create a flat or angled surface for the implant to mate with.

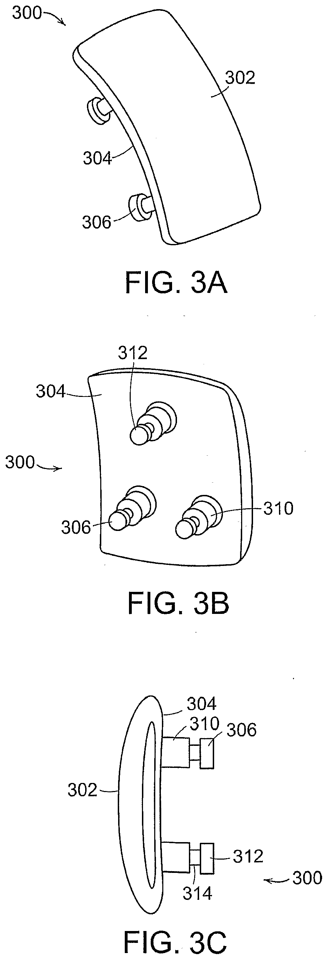

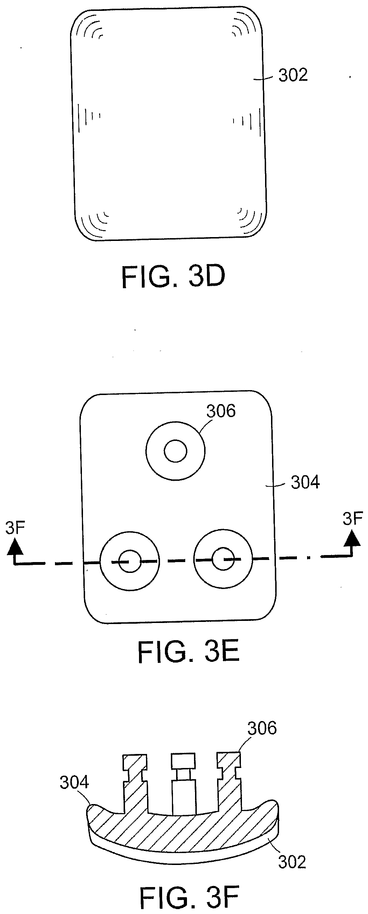

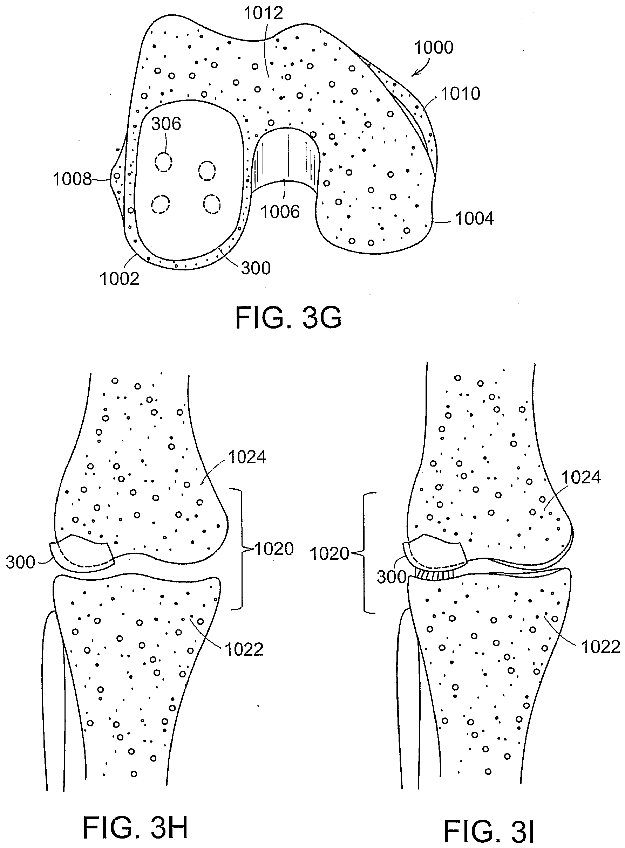

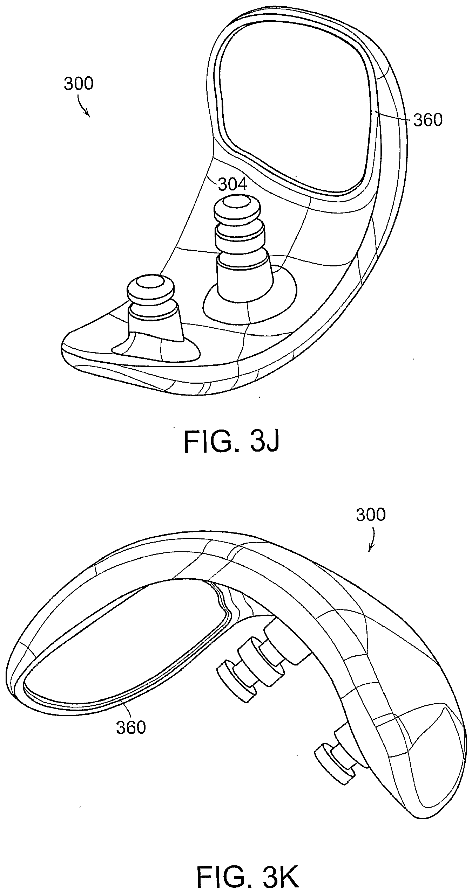

**FIGS. 3A** and B are perspective views of a joint implant suitable for use on a condyle of the femur from the inferior and superior surface viewpoints, respectively. **FIG. 3C** is a side view of the implant of **FIG. 3A****.** **FIG. 3D** is a view of the inferior surface of the implant; **FIG. 3E** is a view of the superior surface of the implant and **FIG. 3F** is a cross-section of the implant. **FIG. 3G** is an axial view of a femur with the implant installed thereon. **FIG. 3H** is an anterior view of the knee joint without the patella wherein the implant is installed on the femoral condyle. **FIG. 3I** is an anterior view of the knee joint with an implant of **FIG. 3A** implanted on the femoral condyle along with an implant suitable for the tibial plateau, such as that shown in **FIG. 2****.** **FIGS. 3J-K** illustrate an alternate embodiment of a joint implant for use on a condyle of a femur further having at least one chamfer cut.

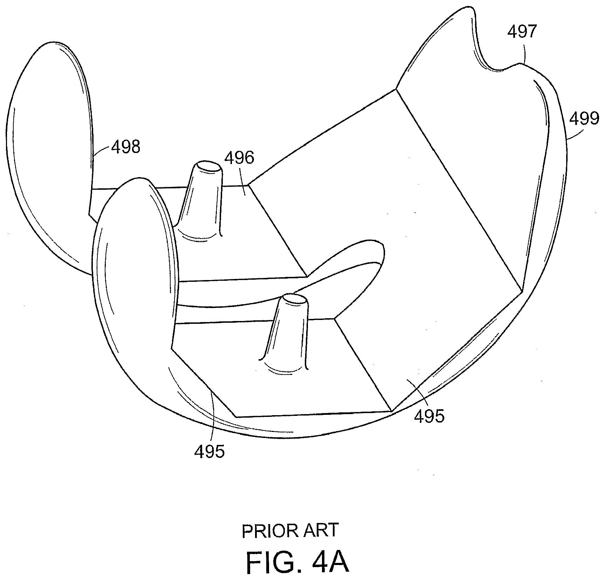

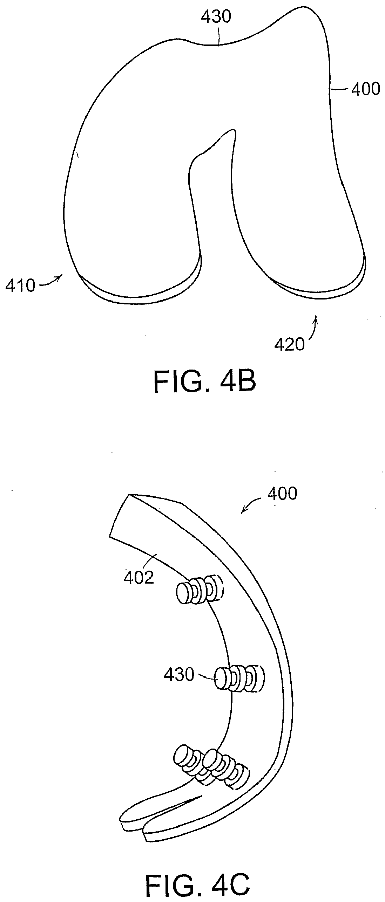

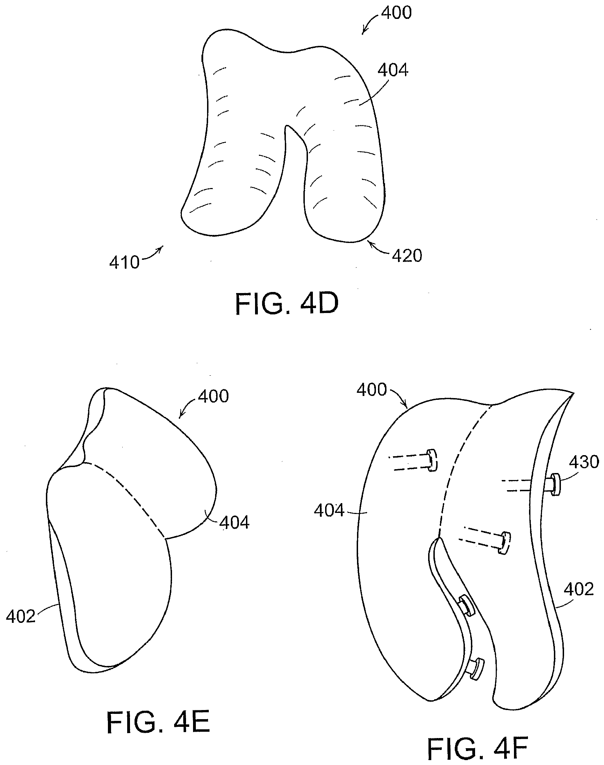

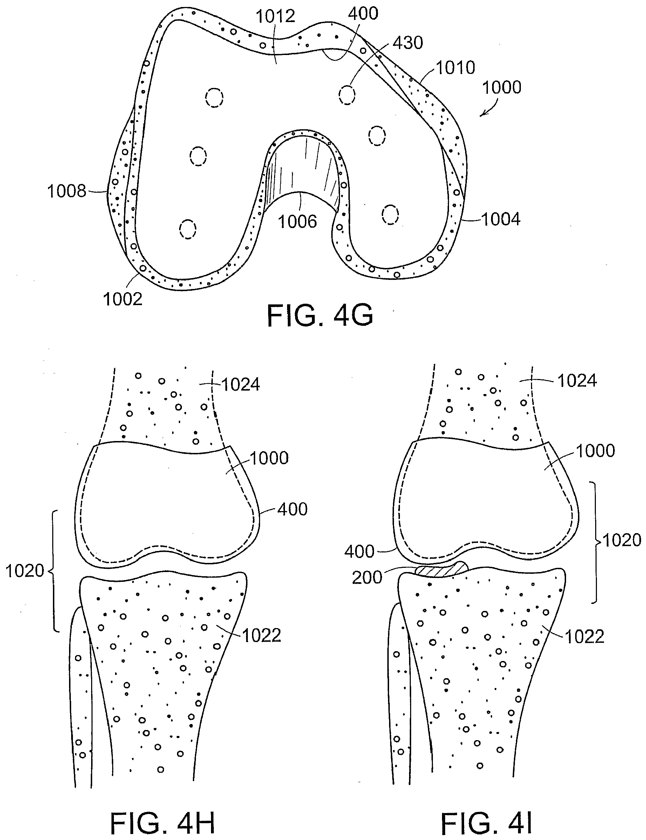

**FIG. 4A** illustrates an implant suitable for the femoral condyle according to the prior art. **FIGS. 4B-I** depict another implant suitable for placement on a femoral condyle. **FIG. 4B** is a slightly perspective view of the implant from the superior surface. **FIG. 4C** is a side view of the implant of **FIG. 4B****.** **FIG. 4D** is a top view of the inferior surface of the implant; **FIG. 4E** and **F** are perspective side views of the implant. **FIG. 4G** is an axial view of a femur with the implant installed thereon. **FIG. 4H** is an anterior view of the knee joint without the patella wherein the implant is installed on the femoral condyle. **FIG. 4I** is an anterior view of the knee joint with an implant of **FIG. 4B** implanted on the femoral condyle along with an implant suitable for the tibial plateau, such as that shown in **FIG. 2****.**

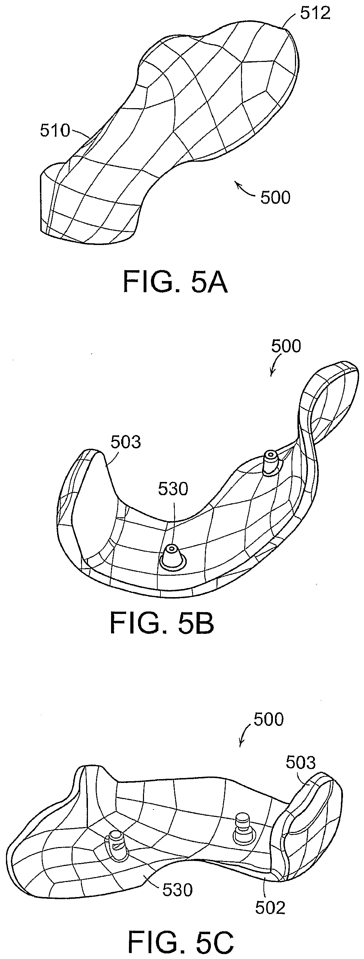

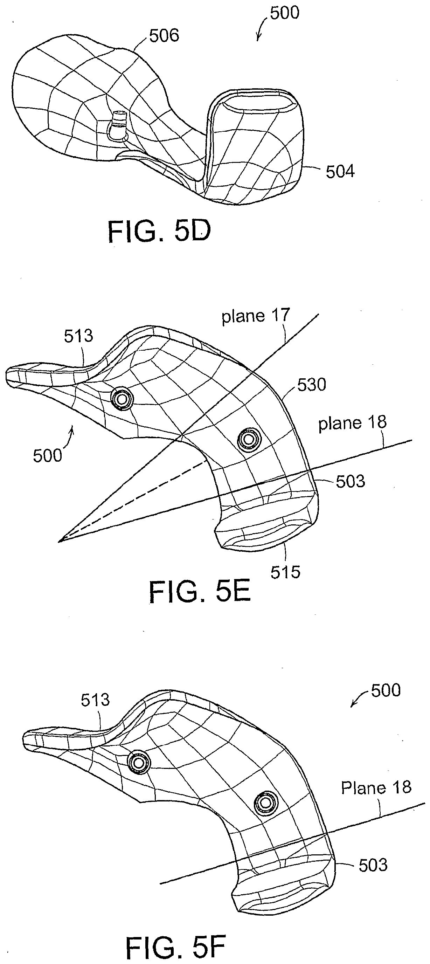

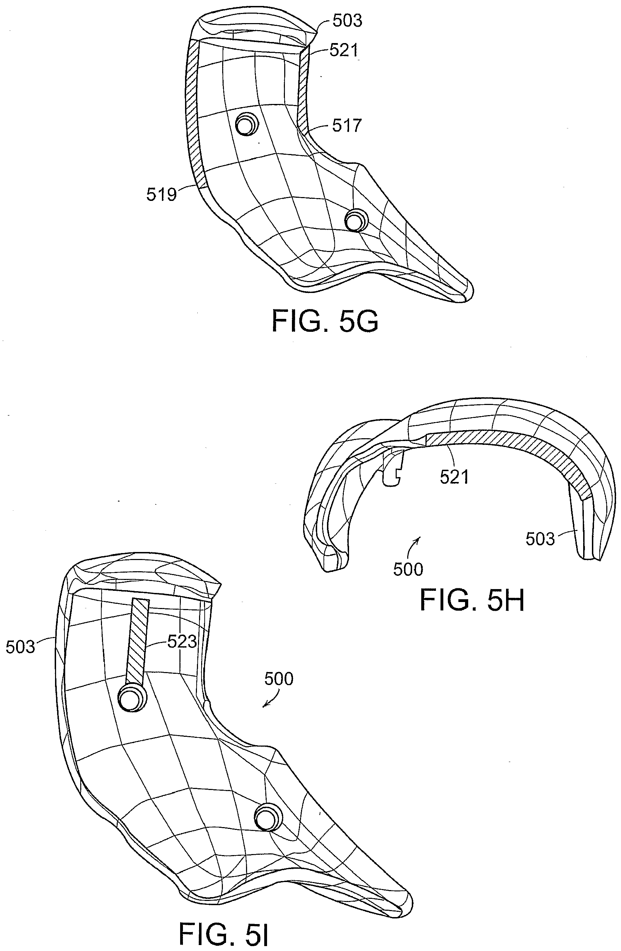

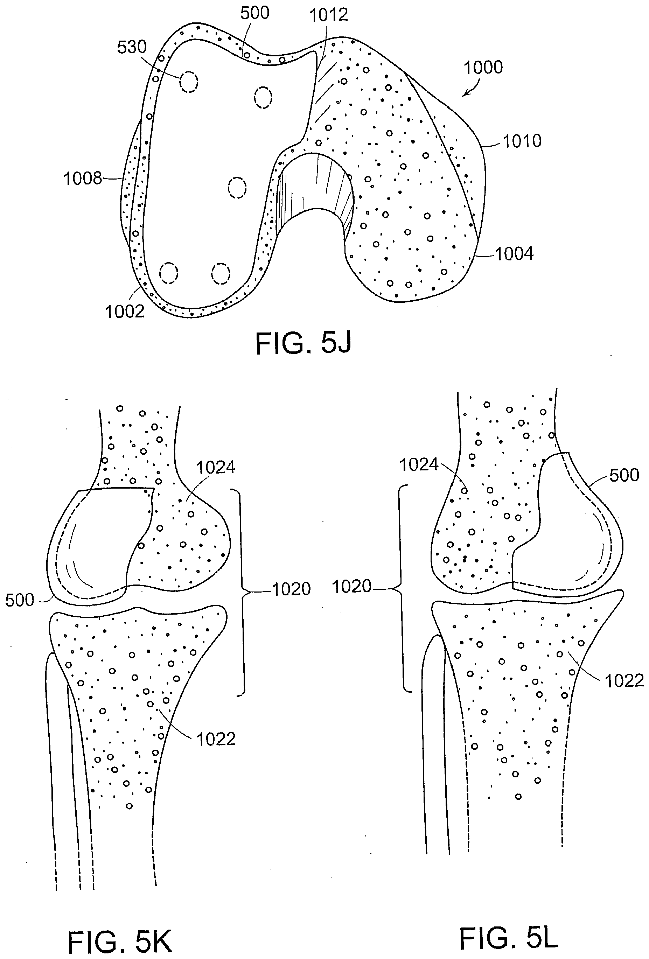

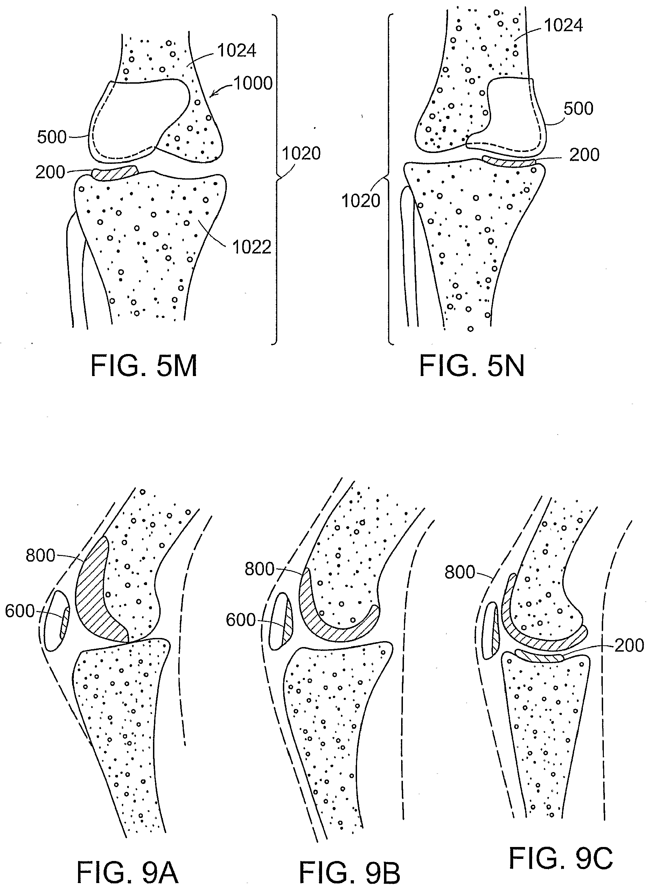

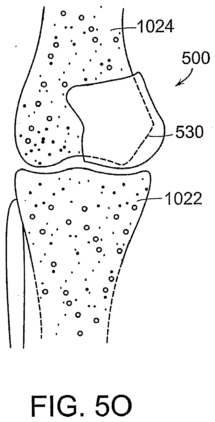

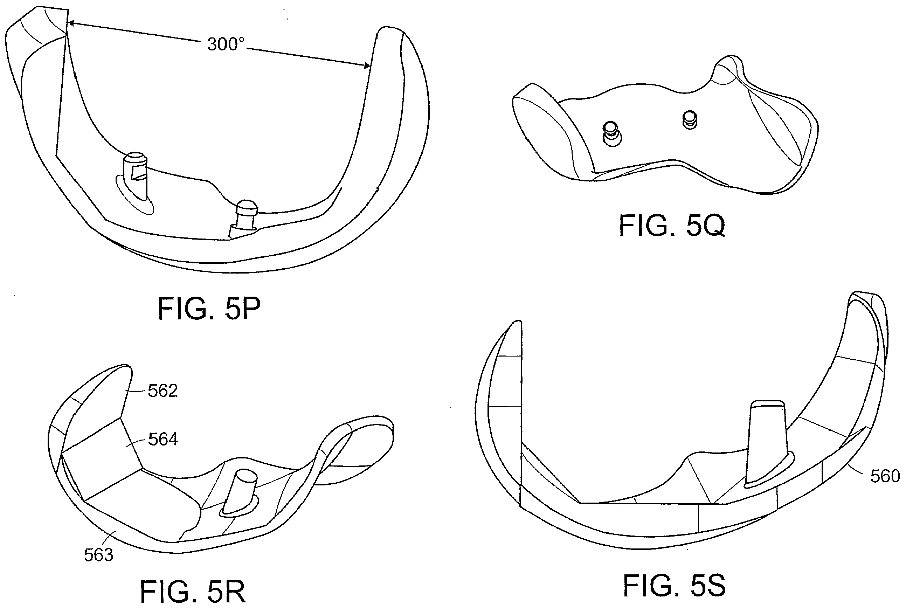

**FIGS. 5A-S** are depictions of another implant suitable for placement on the femoral condyle. **FIG. 5A** is a top view of the inferior surface of the implant showing a chamfer cut. **FIG. 5B** is a slightly perspective view of the superior surface of the implant. **FIG. 5C** is a perspective side view of the implant from a first direction; **FIG. 5D** is a slightly perspective side view of the implant from a second direction. **FIGS. 5E-F** are side views of the implant showing the bearing loads; **FIGS. 5G** and **H** illustrate an alternative embodiment wherein the implant has lateral rails; **FIG. 5I** illustrates another embodiment wherein the implant has an anchoring keel. **FIG. 5J** is an axial view of a femur with the implant installed on the femoral condyles. **FIG. 5K** is an anterior view of the knee joint without the patella wherein the implant is installed on the femoral condyle. **FIG. 5L** is an anterior view of the knee joint with an implant of **FIG. 5A** implanted on the femoral condyles along with an implant suitable for the tibial plateau, such as that shown in **FIG. 2****.** **FIGS. 5M-N** depicts a device implanted within the knee joint. **FIG. 5O** depicts an alternate embodiment of the device which accommodates an partial removal of the condyle. **FIGS. 5P-S** illustrate alternative embodiments of the implant having one or more chamfer cuts.
**FIGS. 6A-G** illustrate a device as shown in **FIG.** 5 along with a graphical representation of the cross-sectional data points comprising the surface map.
**FIGS. 7A-C** illustrate an alternate design of a device, suitable for a portion of the femoral condyle, having a two piece configuration.
**FIGS. 8A-J** depict a whole patella (**FIG. 8A**) and a patella that has been cut in order to install an implant (**FIG. 8B**). A top and side view of a suitable patella implant is shown **(****FIGS. 8C-D**), and an illustration of the implant superimposed on a whole patella is shown to illustrate the location of the implant dome relative to the patellar ridge. **FIGS. 8E-F** illustrate the implant superimposed over a patella. **FIGS. 8G-J** illustrate an alternate design for the patella implant based on a blank (**FIG. 8G**).
**FIGS. 9A-C** depict representative side views of a knee joint with any of the devices taught installed therein. **FIG. 9A** depicts the knee with a condyle implant and a patella implant. **FIG. 9B** depicts an alternate view of the knee with a condyle implant and a patella implant wherein the condyle implant covers a greater portion of the surface of the condyle in the posterior direction. **FIG. 9C** illustrates a knee joint wherein the implant is provided on the condyle, the patella and the tibial plateau.
**FIGS. 10A-D** depict a frontal view of the knee joint with any of the devices taught installed therein. **FIG. 10A** depicts the knee with a tibial implant. **FIG. 10B** depicts the knee with a condyle implant. **FIG. 10C** depicts a knee with a tibial implant and a condyle implant. **FIG. 10C** depicts a knee with a bicompartmental condyle implant and a tibial implant.
**FIG. 11A** shows a joint implant that includes a mobile bearing, in accordance with one embodiment of the invention. **FIGS. 11B-K** show exemplary external surfaces of the joint implant (e.g., facing the femur in a knee implant), in accordance with various embodiments of the invention.
**FIGS. 12A-E** show anchoring mechanisms for a joint implant that includes a mobile bearing, in accordance with various embodiments of the invention.
**FIGS. 13A-G** show bearing surfaces for a joint implant that includes a mobile bearing, in accordance with various embodiments of the invention.
**FIGS. 14A-N** shows perimeter, keel and peg configurations for the first component of an implant that includes a mobile bearing, in accordance with various embodiments of the invention.
**FIGS. 15A-D** show top surface radius configurations for a mobile bearing joint implant, in accordance with various embodiments of the invention.
**FIGS. 16A-J** show bearing and other surface configurations for a mobile bearing joint implant, in accordance with various embodiments of the invention.
**Figs. 17A-D** show mobile bearing joint implant wherein the bearing component of the joint implant is slideably engaged with the first component, in accordance with various embodiments of the invention. **FIGS. 17E-L** show exemplary locations and configurations of the recessed slot of the first component, in accordance with various embodiments of the invention.
**FIGS. 18A-F** show mobile bearing joint devices having a stop restricting motion of the bearing component in one or more dimensions, in accordance with various embodiments of the invention.
**FIGS. 19A-E** show mobile bearing joint implant that include a stop, in accordance with various embodiments of the invention.
**FIGS. 20A-C** show varying shapes of bearing and first components of a mobile bearing joint implant, in accordance with various embodiments of the invention.

### Detailed Description of Specific Embodiments

The following description is presented to enable any person skilled in the art to make and use the invention. Various modifications to the embodiments described will be readily apparent to those skilled in the art, and the generic principles defined herein can be applied to other embodiments and applications without departing from the spirit and scope of the present invention as defined by the appended claims. Thus, the present invention is not intended to be limited to the embodiments shown, but is to be accorded the widest scope consistent with the principles and features disclosed herein. To the extent necessary to achieve a complete understanding of the invention disclosed, the specification and drawings of all issued patents, patent publications, and patent applications cited in this application are incorporated herein by reference.

As will be appreciated by those of skill in the art, methods recited herein may be carried out in any order of the recited events which is logically possible, as well as the recited order of events. Furthermore, where a range of values is provided, it is understood that every intervening value, between the upper and lower limit of that range and any other stated or intervening value in that stated range is encompassed within the invention. Also, it is contemplated that any optional feature of the inventive variations described may be set forth and claimed independently, or in combination with any one or more of the features described herein.

The practice of the present invention can employ, unless otherwise indicated, conventional and digital methods of x-ray imaging and processing, x-ray tomosynthesis, ultrasound including A-scan, B-scan and C-scan, computed tomography (CT scan), magnetic resonance imaging (MRI), optical coherence tomography, single photon emission tomography (SPECT) and positron emission tomography (PET) within the skill of the art. Such techniques are explained fully in the literature and need not be described herein. See, *e.g.,* X-Ray Structure Determination: A Practical Guide, 2nd Edition, editors Stout and Jensen, 1989, John Wiley & Sons, publisher; Body CT: A Practical Approach, editor Slone, 1999, McGraw-Hill publisher; X-ray Diagnosis: A Physician's Approach, editor Lam, 1998 Springer-Verlag, publisher; and Dental Radiology: Understanding the X-Ray Image, editor Laetitia Brocklebank 1997, Oxford University Press publisher. *See also,* The Essential Physics of Medical Imaging (2nd Ed.), Jerrold T. Bushberg, et al.

The present invention provides methods and compositions for repairing joints, particularly for repairing articular cartilage and for facilitating the integration of a wide variety of cartilage repair materials into a subject. Among other things, the techniques described herein allow for the customization of cartilage repair material to suit a particular subject, for example in terms of size, cartilage thickness and/or curvature. When the shape (*e.g*., size, thickness and/or curvature) of the articular cartilage surface is an exact or near anatomic fit with the non-damaged cartilage or with the subject's original cartilage, the success of repair is enhanced. The repair material can be shaped prior to implantation and such shaping can be based, for example, on electronic images that provide information regarding curvature or thickness of any "normal" cartilage surrounding the defect and/or on curvature of the bone underlying the defect. Thus, the current invention provides, among other things, for minimally invasive methods for partial joint replacement. The methods will require only minimal or, in some instances, no loss in bone stock. Additionally, unlike with current techniques, the methods described herein will help to restore the integrity of the articular surface by achieving an exact or near anatomic match between the implant and the surrounding or adjacent cartilage and/or subchondral bone.

Advantages of the present invention can include, but are not limited to, (i) customization of joint repair, thereby enhancing the efficacy and comfort level for the patient following the repair procedure; (ii) eliminating the need for a surgeon to measure the defect to be repaired intraoperatively in some embodiments; (iii) eliminating the need for a surgeon to shape the material during the implantation procedure; (iv) providing methods of evaluating curvature of the repair material based on bone or tissue images or based on intraoperative probing techniques; (v) providing methods of repairing joints with only minimal or, in some instances, no loss in bone stock; (vi) improving postoperative joint congruity; (vii) improving the postoperative patient recovery in some embodiments and (viii) improving postoperative function, such as range of motion.

Thus, the methods described herein allow for the design and use of joint repair material that more precisely fits the defect (*e.g*., site of implantation) or the articular surface(s) and, accordingly, provides improved repair of the joint.

### I. ASSESSMENT OF JOINTS AND ALIGNMENT

The methods and compositions described herein can be used to treat defects resulting from disease of the cartilage (*e.g*., osteoarthritis), bone damage, cartilage damage, trauma, and/or degeneration due to overuse or age. The invention allows, among other things, a health practitioner to evaluate and treat such defects. The size, volume and shape of the area of interest can include only the region of cartilage that has the defect, but preferably will also include contiguous parts of the cartilage surrounding the cartilage defect.

As will be appreciated by those of skill in the art, size, curvature and/or thickness measurements can be obtained using any suitable technique. For example, one-dimensional, two-dimensional, and/or three-dimensional measurements can be obtained using suitable mechanical means, laser devices, electromagnetic or optical tracking systems, molds, materials applied to the articular surface that harden and "memorize the surface contour," and/or one or more imaging techniques known in the art. Measurements can be obtained non-invasively and/or intraoperatively (e.g., using a probe or other surgical device). As will be appreciated by those of skill in the art, the thickness of the repair device can vary at any given point depending upon patient's anatomy and/or the depth of the damage to the cartilage and/or bone to be corrected at any particular location on an articular surface.

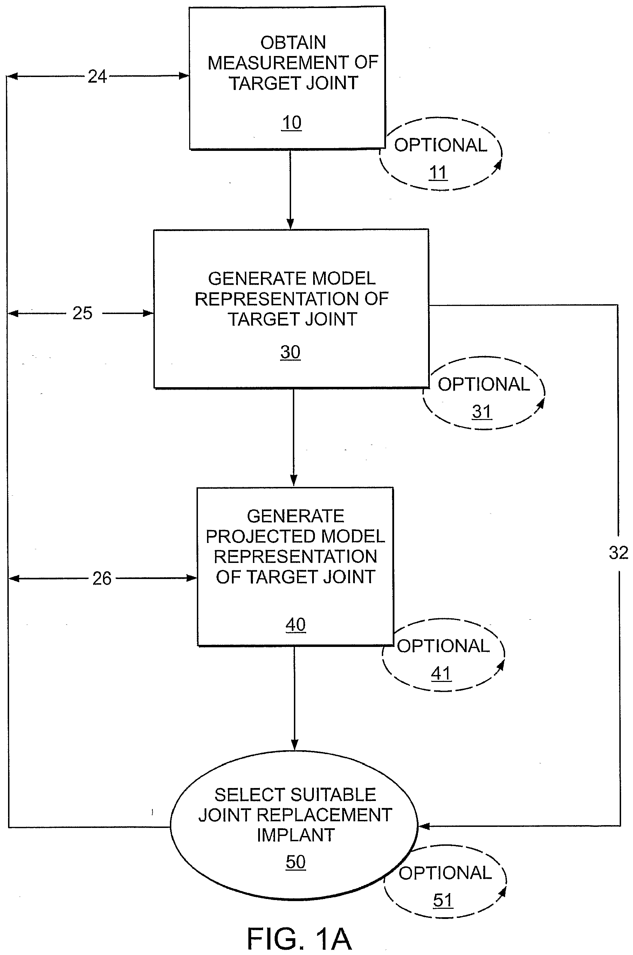

**FIG. 1A** is a flow chart showing steps taken by a practitioner in assessing a joint. First, a practitioner obtains a measurement of a target joint ***10.*** The step of obtaining a measurement can be accomplished by taking an image of the joint. This step can be repeated, as necessary, ***11*** to obtain a plurality of images in order to further refine the joint assessment process. Once the practitioner has obtained the necessary measurements, the information is used to generate a model representation of the target joint being assessed ***30.*** This model representation can be in the form of a topographical map or image. The model representation of the joint can be in one, two, or three dimensions. It can include a physical model. More than one model can be created ***31,*** if desired. Either the original model, or a subsequently created model, or both can be used. After the model representation of the joint is generated ***30,*** the practitioner can optionally generate a projected model representation of the target joint in a corrected condition ***40,*** e.g., from the existing cartilage on the joint surface, by providing a mirror of the opposing joint surface, or a combination thereof Again, this step can be repeated ***41,*** as necessary or desired. Using the difference between the topographical condition of the joint and the projected image of the joint, the practitioner can then select a joint implant ***50*** that is suitable to achieve the corrected joint anatomy. As will be appreciated by those of skill in the art, the selection process ***50*** can be repeated ***51*** as often as desired to achieve the desired result. Additionally, it is contemplated that a practitioner can obtain a measurement of a target joint ***10*** by obtaining, for example, an x-ray, and then select a suitable joint replacement implant ***50.***

As will be appreciated by those of skill in the art, the practitioner can proceed directly from the step of generating a model representation of the target joint ***30*** to the step of selecting a suitable joint replacement implant ***50*** as shown by the arrow ***32.*** Additionally, following selection of suitable joint replacement implant ***50,*** the steps of obtaining measurement of target joint ***10,*** generating model representation of target joint ***30*** and generating projected model ***40,*** can be repeated in series or parallel as shown by the flow ***24, 25, 26.***

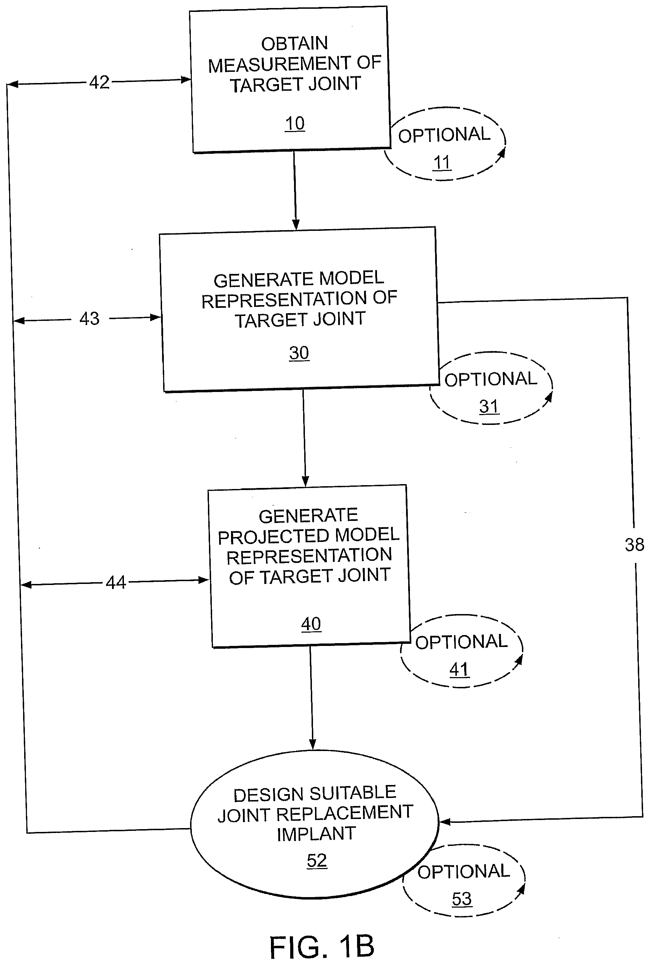

**FIG. 1B** is an alternate flow chart showing steps taken by a practitioner in assessing a joint. First, a practitioner obtains a measurement of a target joint ***10**.* The step of obtaining a measurement can be accomplished by taking an image of the joint. This step can be repeated, as necessary, ***11*** to obtain a plurality of images in order to further refine the joint assessment process. Once the practitioner has obtained the necessary measurements, the information is used to generate a model representation of the target joint being assessed ***30**.* This model representation can be in the form of a topographical map or image. The model representation of the joint can be in one, two, or three dimensions. The process can be repeated ***31*** as necessary or desired. It can include a physical model. After the model representation of the joint is assessed ***30,*** the practitioner can optionally generate a projected model representation of the target joint in a corrected condition ***40**.* This step can be repeated ***41*** as necessary or desired. Using the difference between the topographical condition of the joint and the projected image of the joint, the practitioner can then design a joint implant ***52*** that is suitable to achieve the corrected joint anatomy, repeating the design process ***53*** as often as necessary to achieve the desired implant design. The practitioner can also assess whether providing additional features, such as rails, keels, lips, pegs, cruciate stems, or anchors, cross-bars, etc. will enhance the implants' performance in the target joint.

As will be appreciated by those of skill in the art, the practitioner can proceed directly from the step of generating a model representation of the target joint ***30*** to the step of designing a suitable joint replacement implant ***52*** as shown by the arrow ***38.*** Similar to the flow shown above, following the design of a suitable joint replacement implant ***52,*** the steps of obtaining measurement of target joint ***10,*** generating model representation of target joint 30 and generating projected model ***40,*** can be repeated in series or parallel as shown by the flow ***42, 43, 44.***

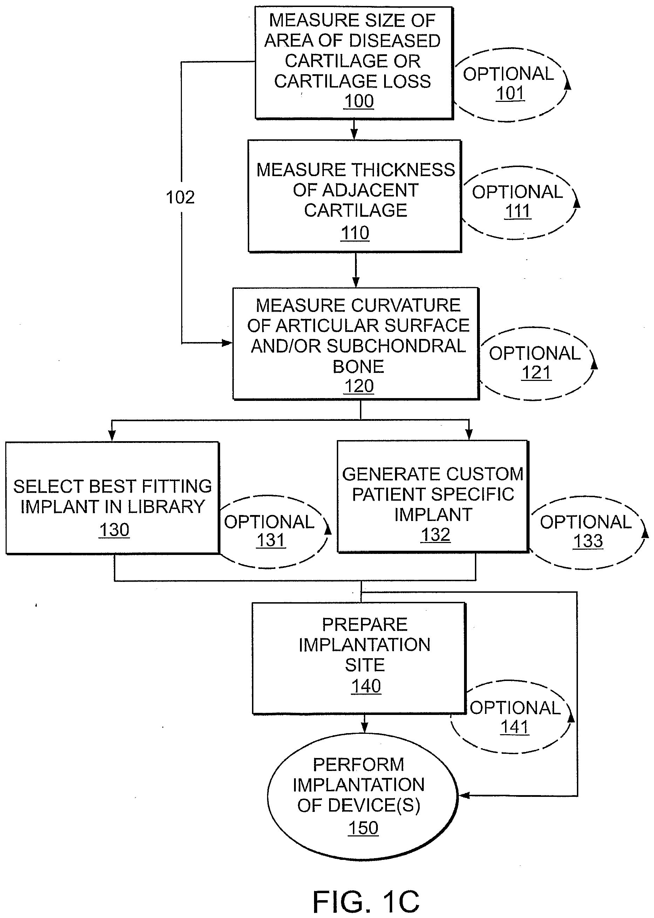

**FIG. 1C** is a flow chart illustrating the process of selecting an implant for a patient. First, using the techniques described above or those suitable and known in the art at the time the invention is practiced, the size of area of diseased cartilage or cartilage loss is measured ***100.*** This step can be repeated multiple times ***101,*** as desired. Once the size of the cartilage defect is measured, the thickness of adjacent cartilage can optionally be measured ***110.*** This process can also be repeated as desired ***111.*** Either after measuring the cartilage loss or measuring the thickness of adjacent cartilage, the curvature of the articular surface is then measured ***120.*** Alternatively, the subchondral bone can be measured. As will be appreciated measurements can be taken of the surface of the joint being repaired, or of the mating surface in order to facilitate development of the best design for the implant surface.

Once the surfaces have been measured, the user either selects the best fitting implant contained in a library of implants ***130*** or generates a patient-specific implant ***132.*** These steps can be repeated as desired or necessary to achieve the best fitting implant for a patient, ***131, 133.*** As will be appreciated by those of skill in the art, the process of selecting or designing an implant can be tested against the information contained in the MRI or x-ray of the patient to ensure that the surfaces of the device achieves a good fit relative to the patient's joint surface. Testing can be accomplished by, for example, superimposing the implant image over the image for the patient's joint. Once it has been determined that a suitable implant has been selected or designed, the implant site can be prepared ***140,*** for example by removing cartilage or bone from the joint surface, or the implant can be placed into the joint ***150.***

The joint implant selected or designed achieves anatomic or near anatomic fit with the existing surface of the joint while presenting a mating surface for the opposing joint surface that replicates the natural joint anatomy. In this instance, both the existing surface of the joint can be assessed as well as the desired resulting surface of the joint. This technique is particularly useful for implants that are not anchored into the bone.

As will be appreciated by those of skill in the art, the physician, or other person practicing the invention, can obtain a measurement of a target joint 10 and then either design 52 or select ***50*** a suitable joint replacement implant.

### II. REPAIR MATERIALS

A wide variety of materials find use in the practice of the present invention, including, but not limited to, plastics, metals, crystal free metals, ceramics, biological materials (*e.g.,* collagen or other extracellular matrix materials), hydroxyapatite, cells (*e.g.,* stem cells, chondrocyte cells or the like), or combinations thereof. Based on the information (*e.g.,* measurements) obtained regarding the defect and the articular surface and/or the subchondral bone, a repair material can be formed or selected. Further, using one or more of these techniques described herein, a cartilage replacement or regenerating material having a curvature that will fit into a particular cartilage defect, will follow the contour and shape of the articular surface, and will match the thickness of the surrounding cartilage. The repair material can include any combination of materials, and typically includes at least one non-pliable material, for example materials that are not easily bent or changed.

### A. METAL AND POLYMERIC REPAIR MATERIALS

Currently, joint repair systems often employ metal and/or polymeric materials including, for example, prostheses which are anchored into the underlying bone (*e.g.,* a femur in the case of a knee prosthesis). See, *e.g.,* U.S. Patent No. 6,203,576 to Afriat, et al. issued March 20, 2001 and 6,322,588 to Ogle, et al. issued November 27, 2001, and references cited therein. A wide-variety of metals are useful in the practice of the present invention, and can be selected based on any criteria. For example, material selection can be based on resiliency to impart a desired degree of rigidity. Non-limiting examples of suitable metals include silver, gold, platinum, palladium, iridium, copper, tin, lead, antimony, bismuth, zinc, titanium, cobalt, stainless steel, nickel, iron alloys, cobalt alloys, such as Elgiloy®, a cobalt-chromium-nickel alloy, and MP35N, a nickel-cobalt-chromium-molybdenum alloy, and Nitinol™, a nickel-titanium alloy, aluminum, manganese, iron, tantalum, crystal free metals, such as Liquidmetal® alloys (available from LiquidMetal Technologies, www.liquidmetal.com), other metals that can slowly form polyvalent metal ions, for example to inhibit calcification of implanted substrates in contact with a patient's bodily fluids or tissues, and combinations thereof.

Suitable synthetic polymers include, without limitation, polyamides (*e.g.,* nylon), polyesters, polystyrenes, polyacrylates, vinyl polymers (*e.g.,* polyethylene, polytetrafluoroethylene, polypropylene and polyvinyl chloride), polycarbonates, polyurethanes, poly dimethyl siloxanes, cellulose acetates, polymethyl methacrylates, polyether ether ketones, ethylene vinyl acetates, polysulfones, nitrocelluloses, similar copolymers and mixtures thereof. Bioresorbable synthetic polymers can also be used such as dextran, hydroxyethyl starch, derivatives of gelatin, polyvinylpyrrolidone, polyvinyl alcohol, poly[N-(2-hydroxypropyl) methacrylamide], poly(hydroxy acids), poly(epsilon-caprolactone), polylactic acid, polyglycolic acid, poly(dimethyl glycolic acid), poly(hydroxy butyrate), and similar copolymers can also be used.

Other materials would also be appropriate, for example, the polyketone known as polyetheretherketone (PEEK™). This includes the material PEEK 450G, which is an unfilled PEEK approved for medical implantation available from Victrex of Lancashire, Great Britain. (Victrex is located at www.matweb.com or see Boedeker www.boedeker.com). Other sources of this material include Gharda located in Panoli, India (www.ghardapolymers.com).

It should be noted that the material selected can also be filled. For example, other grades of PEEK are also available and contemplated, such as 30% glass-filled or 30% carbon filled, provided such materials are cleared for use in implantable devices by the FDA, or other regulatory body. Glass filled PEEK reduces the expansion rate and increases the flexural modulus of PEEK relative to that portion which is unfilled. The resulting product is known to be ideal for improved strength, stiffness, or stability. Carbon filled PEEK is known to enhance the compressive strength and stiffness of PEEK and lower its expansion rate. Carbon filled PEEK offers wear resistance and load carrying capability.

As will be appreciated, other suitable similarly biocompatible thermoplastic or thermoplastic polycondensate materials that resist fatigue, have good memory, are flexible, and/or deflectable have very low moisture absorption, and good wear and/or abrasion resistance, can be used without departing from the scope of the invention. The implant can also be comprised of polyetherketoneketone (PEKK).

Other materials that can be used include polyetherketone (PEK), polyetherketoneetherketoneketone (PEKEKK), and polyetheretherketoneketone (PEEKK), and generally a polyaryletheretherketone. Further other polyketones can be used as well as other thermoplastics.

Reference to appropriate polymers that can be used for the implant can be made to the following documents, all of which are incorporated herein by reference. These documents include: PCT Publication WO 02/02158 A1, dated Jan. 10, 2002 and entitled Bio-Compatible Polymeric Materials; PCT Publication WO 02/00275 A1, dated Jan. 3, 2002 and entitled Bio-Compatible Polymeric Materials; and PCT Publication WO 02/00270 A1, dated Jan. 3, 2002 and entitled Bio-Compatible Polymeric Materials.

The polymers can be prepared by any of a variety of approaches including conventional polymer processing methods. Preferred approaches include, for example, injection molding, which is suitable for the production of polymer components with significant structural features, and rapid prototyping approaches, such as reaction injection molding and stereo-lithography. The substrate can be textured or made porous by either physical abrasion or chemical alteration to facilitate incorporation of the metal coating. Other processes are also appropriate, such as extrusion, injection, compression molding and/or machining techniques. Typically, the polymer is chosen for its physical and mechanical properties and is suitable for carrying and spreading the physical load between the joint surfaces.

More than one metal and/or polymer can be used in combination with each other. For example, one or more metal-containing substrates can be coated with polymers in one or more regions or, alternatively, one or more polymer-containing substrate can be coated in one or more regions with one or more metals.

The system or prosthesis can be porous or porous coated. The porous surface components can be made of various materials including metals, ceramics, and polymers. These surface components can, in turn, be secured by various means to a multitude of structural cores formed of various metals. Suitable porous coatings include, but are not limited to, metal, ceramic, polymeric (*e.g.,* biologically neutral elastomers such as silicone rubber, polyethylene terephthalate and/or combinations thereof) or combinations thereof. *See, e.g.,* U.S. Pat. No. 3,605,123 to Hahn, issued September 20, 1971. U.S. Pat. No. 3,808,606 to Tronzo issued May 7, 1974 and U.S. Pat. No. 3,843,975 to Tronzo issued October 29, 1974; U.S. Pat. No. 3,314,420 to Smith issued April 18, 1967; U.S. Pat. No. 3,987,499 to Scharbach issued October 26, 1976; and German Offenlegungsschrift 2,306,552. There can be more than one coating layer and the layers can have the same or different porosities. See, *e.g.,* U.S. Pat. No. 3,938,198 to Kahn, et al., issued February 17, 1976.

The coating can be applied by surrounding a core with powdered polymer and heating until cured to form a coating with an internal network of interconnected pores. The tortuosity of the pores (*e.g.,* a measure of length to diameter of the paths through the pores) can be important in evaluating the probable success of such a coating in use on a prosthetic device. See, also, U.S. Pat. No. 4,213,816 to Morris issued July 22, 1980. The porous coating can be applied in the form of a powder and the article as a whole subjected to an elevated temperature that bonds the powder to the substrate. Selection of suitable polymers and/or powder coatings can be determined in view of the teachings and references cited herein, for example based on the melt index of each.

### B. BIOLOGICAL REPAIR MATERIAL

Repair materials can also include one or more biological material either alone or in combination with non-biological materials. For example, any base material can be designed or shaped and suitable cartilage replacement or regenerating material(s) such as fetal cartilage cells can be applied to be the base. The cells can be then be grown in conjunction with the base until the thickness (and/or curvature) of the cartilage surrounding the cartilage defect has been reached. Conditions for growing cells (*e.g.,* chondrocytes) on various substrates in culture, *ex vivo* and *in vivo* are described, for example, in U.S. Patent Nos. 5,478,739 to Slivka et al. issued December 26, 1995; 5,842,477 to Naughton et al. issued December 1, 1998; 6,283,980 to Vibe-Hansen et al., issued September 4, 2001, and 6,365,405 to Salzmann et al. issued April 2, 2002. Non-limiting examples of suitable substrates include plastic, tissue scaffold, a bone replacement material (*e.g.,* a hydroxyapatite, a bioresorbable material), or any other material suitable for growing a cartilage replacement or regenerating material on it.

Biological polymers can be naturally occurring or produced *in vitro* by fermentation and the like. Suitable biological polymers include, without limitation, collagen, elastin, silk, keratin, gelatin, polyamino acids, cat gut sutures, polysaccharides (*e.g.,* cellulose and starch) and mixtures thereof. Biological polymers can be bioresorbable.

Biological materials used in the methods described herein can be autografts (from the same subject); allografts (from another individual of the same species) and/or xenografts (from another species). See, also, International Patent Publications WO 02/22014 to Alexander et al. published March 21, 2002 and WO 97/27885 to Lee published August 7, 1997. In certain embodiments autologous materials are preferred, as they can carry a reduced risk of immunological complications to the host, including reabsorption of the materials, inflammation and/or scarring of the tissues surrounding the implant site.

In one embodiment of the invention, a probe is used to harvest tissue from a donor site and to prepare a recipient site. The donor site can be located in a xenograft, an allograft or an autograft. The probe is used to achieve a good anatomic match between the donor tissue sample and the recipient site. The probe is specifically designed to achieve a seamless or near seamless match between the donor tissue sample and the recipient site. The probe can, for example, be cylindrical. The distal end of the probe is typically sharp in order to facilitate tissue penetration. Additionally, the distal end of the probe is typically hollow in order to accept the tissue. The probe can have an edge at a defined distance from its distal end, *e.g.* at 1 cm distance from the distal end and the edge can be used to achieve a defined depth of tissue penetration for harvesting. The edge can be external or can be inside the hollow portion of the probe. For example, an orthopedic surgeon can take the probe and advance it with physical pressure into the cartilage, the subchondral bone and the underlying marrow in the case of a joint such as a knee joint. The surgeon can advance the probe until the external or internal edge reaches the cartilage surface. At that point, the edge will prevent further tissue penetration thereby achieving a constant and reproducible tissue penetration. The distal end of the probe can include one or more blades, saw-like structures, or tissue cutting mechanism. For example, the distal end of the probe can include an iris-like mechanism consisting of several small blades. The blade or blades can be moved using a manual, motorized or electrical mechanism thereby cutting through the tissue and separating the tissue sample from the underlying tissue. Typically, this will be repeated in the donor and the recipient. In the case of an iris-shaped blade mechanism, the individual blades can be moved so as to close the iris thereby separating the tissue sample from the donor site.

In another embodiment of the invention, a laser device or a radiofrequency device can be integrated inside the distal end of the probe. The laser device or the radiofrequency device can be used to cut through the tissue and to separate the tissue sample from the underlying tissue.

In one embodiment of the invention, the same probe can be used in the donor and in the recipient. In another embodiment, similarly shaped probes of slightly different physical dimensions can be used. For example, the probe used in the recipient can be slightly smaller than that used in the donor thereby achieving a tight fit between the tissue sample or tissue transplant and the recipient site. The probe used in the recipient can also be slightly shorter than that used in the donor thereby correcting for any tissue lost during the separation or cutting of the tissue sample from the underlying tissue in the donor material.

Any biological repair material can be sterilized to inactivate biological contaminants such as bacteria, viruses, yeasts, molds, mycoplasmas and parasites. Sterilization can be performed using any suitable technique, for example radiation, such as gamma radiation.

Any of the biological materials described herein can be harvested with use of a robotic device. The robotic device can use information from an electronic image for tissue harvesting.

In certain embodiments, the cartilage replacement material has a particular biochemical composition. For instance, the biochemical composition of the cartilage surrounding a defect can be assessed by taking tissue samples and chemical analysis or by imaging techniques. For example, WO 02/22014 to Alexander describes the use of gadolinium for imaging of articular cartilage to monitor glycosaminoglycan content within the cartilage. The cartilage replacement or regenerating material can then be made or cultured in a manner, to achieve a biochemical composition similar to that of the cartilage surrounding the implantation site. The culture conditions used to achieve the desired biochemical compositions can include, for example, varying concentrations. Biochemical composition of the cartilage replacement or regenerating material can, for example, be influenced by controlling concentrations and exposure times of certain nutrients and growth factors.

### III. DEVICE DESIGN

Using information on thickness and curvature of the cartilage and/or subchondral bone, a physical model of the surfaces of the articular cartilage and/or of the underlying bone can be created. This physical model can be representative of a limited area within the joint or it can encompass the entire joint. This model can also take into consideration the presence or absence of a meniscus as well as the presence or absence of some or all of the cartilage. For example, in the knee joint, the physical model can encompass only the medial or lateral femoral condyle, both femoral condyles and the notch region, the medial tibial plateau, the lateral tibial plateau, the entire tibial plateau, the medial patella, the lateral patella, the entire patella or the entire joint. The location of a diseased area of cartilage can be determined, for example using a 3D coordinate system or a 3D Euclidian distance as described in WO 02/22014.

In this way, the size of the defect to be repaired can be determined. This process takes into account that, for example, roughly 80% of patients have a healthy lateral component. As will be apparent, some, but not all, defects will include less than the entire cartilage. Thus, in one embodiment of the invention, the thickness of the normal or only mildly diseased cartilage surrounding one or more cartilage defects is measured. This thickness measurement can be obtained at a single point or, preferably, at multiple points, for example 2 point, 4-6 points, 7-10 points, more than 10 points or over the length of the entire remaining cartilage. Furthermore, once the size of the defect is determined, an appropriate therapy (*e.g*., articular repair system) can be selected such that as much as possible of the healthy, surrounding tissue is preserved.

In other embodiments, the curvature of the articular surface can be measured to design and/or shape the repair material. Further, both the thickness of the remaining cartilage and the curvature of the articular surface can be measured to design and/or shape the repair material. Alternatively, the curvature of the subchondral bone can be measured and the resultant measurement(s) can be used to either select or shape a cartilage replacement material. For example, the contour of the subchondral bone can be used to re-create a virtual cartilage surface: the margins of an area of diseased cartilage can be identified. The subchondral bone shape in the diseased areas can be measured. A virtual contour can then be created by copying the subchondral bone surface into the cartilage surface, whereby the copy of the subchondral bone surface connects the margins of the area of diseased cartilage. In shaping the device, the contours can be configured to mate with existing cartilage or to account for the removal of some or all of the cartilage.

**FIG. 2A** shows a slightly perspective top view of a joint implant ***200*** of the invention suitable for implantation at the tibial plateau of the knee joint. As shown in **FIG. 2A****,** the implant can be generated using, for example, a dual surface assessment, as described above with respect to **FIGS. 1A** and **B****.**

The implant ***200*** has an upper surface ***202,*** a lower surface ***204*** and a peripheral edge ***206.*** The upper surface ***202*** is formed so that it forms a mating surface for receiving the opposing joint surface; in this instance partially concave to receive the femur. The concave surface can be variably concave such that it presents a surface to the opposing joint surface, *e.g.* a negative surface of the mating surface of the femur it communicates with. As will be appreciated by those of skill in the art, the negative impression, need not be a perfect one.

The upper surface ***202*** of the implant ***200*** can be shaped by any of a variety of means. For example, the upper surface ***202*** can be shaped by projecting the surface from the existing cartilage and/or bone surfaces on the tibial plateau, or it can be shaped to mirror the femoral condyle in order to optimize the complimentary surface of the implant when it engages the femoral condyle. Alternatively, the superior surface ***202*** can be configured to mate with an inferior surface of an implant configured for the opposing femoral condyle.

The lower surface ***204*** has a convex surface that matches, or nearly matches, the tibial plateau of the joint such that it creates an anatomic or near anatomic fit with the tibial plateau. Depending on the shape of the tibial plateau, the lower surface can be partially convex as well. Thus, the lower surface ***204*** presents a surface to the tibial plateau that fits within the existing surface. It can be formed to match the existing surface or to match the surface after articular resurfacing.

As will be appreciated by those of skill in the art, the convex surface of the lower surface ***204*** need not be perfectly convex. Rather, the lower surface ***204*** is more likely consist of convex and concave portions that fit within the existing surface of the tibial plateau or the re-surfaced plateau. Thus, the surface is essentially variably convex and concave.

**FIG. 2B** shows a top view of the joint implant of **FIG. 2A****.** As shown in **FIG. 2B** the exterior shape ***208*** of the implant can be elongated. The elongated form can take a variety of shapes including elliptical, quasi-elliptical, race-track, etc. However, as will be appreciated the exterior dimension is typically irregular thus not forming a true geometric shape, *e.g.* ellipse. As will be appreciated by those of skill in the art, the actual exterior shape of an implant can vary depending on the nature of the joint defect to be corrected. Thus the ratio of the length ***L*** to the width ***W*** can vary from, for example, between 0.25 to 2.0, and more specifically from 0.5 to 1.5. As further shown in **FIG. 2B****,** the length across an axis of the implant 200 varies when taken at points along the width of the implant. For example, as shown in **FIG. 2B****, L₁ ≠ L₂ ≠ L₃.**

Turning now to **FIGS. 2C-E****,** cross-sections of the implant shown in **FIG. 2B** are depicted along the lines of C-C, D-D, and E-E. The implant has a thickness ***t1, t2*** and ***t3*** respectively. As illustrated by the cross-sections, the thickness of the implant varies along both its length ***L*** and width ***W.*** The actual thickness at a particular location of the implant ***200*** is a function of the thickness of the cartilage and/or bone to be replaced and the joint mating surface to be replicated. Further, the profile of the implant ***200*** at any location along its length ***L*** or width ***W*** is a function of the cartilage and/or bone to be replaced.

**FIG. 2F** is a lateral view of the implant ***200*** of **FIG. 2A****.** In this instance, the height of the implant ***200*** at a first end ***h₁*** is different than the height of the implant at a second end ***h₂.*** Further the upper edge ***208*** can have an overall slope in a downward direction. However, as illustrated the actual slope of the upper edge ***208*** varies along its length and can, in some instances, be a positive slope. Further the lower edge ***210*** can have an overall slope in a downward direction. As illustrated the actual slope of the lower edge ***210*** varies along its length and can, in some instances, be a positive slope. As will be appreciated by those of skill in the art, depending on the anatomy of an individual patient, an implant can be created wherein ***h₁*** and ***h₂*** are equivalent, or substantially equivalent without departing from the scope of the invention.

**FIG. 2G** is a cross-section taken along a sagittal plane in a body showing the implant ***200*** implanted within a knee joint ***1020*** such that the lower surface ***204*** of the implant ***200*** lies on the tibial plateau ***1022*** and the femur ***1024*** rests on the upper surface ***202*** of the implant ***200.*** **FIG. 2H** is a cross-section taken along a coronal plane in a body showing the implant ***200*** implanted within a knee joint ***1020.*** As is apparent from this view, the implant ***200*** is positioned so that it fits within a superior articular surface ***224.*** As will be appreciated by those of skill in the art, the articular surface could be the medial or lateral facet, as needed.

**FIG. 2I** is a view along an axial plane of the body showing the implant ***200*** implanted within a knee joint ***1020*** showing the view taken from an aerial, or upper, view. **FIG. 2J** is a view of an alternate embodiment where the implant is a bit larger such that it extends closer to the bone medially, *i.e.* towards the edge ***1023*** of the tibial plateau, as well as extending anteriorly and posteriorly.

**FIG. 2K** is a cross-section of an implant ***200*** of the invention according to an alternate embodiment. In this embodiment, the lower surface ***204*** further includes a joint anchor ***212.*** As illustrated in this embodiment, the joint anchor ***212*** forms a protrusion, keel or vertical member that extends from the lower surface ***204*** of the implant ***200*** and projects into, for example, the bone of the joint. As will be appreciated by those of skill in the art, the keel can be perpendicular or lie within a plane of the body.

Additionally, as shown in **FIG. 2L** the joint anchor ***212*** can have a cross-member ***214*** so that from a bottom perspective, the joint anchor ***212*** has the appearance of a cross or an "x." As will be appreciated by those of skill in the art, the joint anchor ***212*** could take on a variety of other forms while still accomplishing the same objective of providing increased stability of the implant ***200*** in the joint. These forms include, but are not limited to, pins, bulbs, balls, teeth, etc. Additionally, one or more joint anchors ***212*** can be provided as desired. **FIG. 2M** and **N** illustrate cross-sections of alternate embodiments of a dual component implant from a side view and a front view.

In an alternate embodiment shown in **FIG. 2M** it may be desirable to provide a one or more cross-members ***220*** on the lower surface ***204*** in order to provide a bit of translation movement of the implant relative to the surface of the femur, or femur implant. In that event, the cross-member can be formed integral to the surface of the implant or can be one or more separate pieces that fit within a groove ***222*** on the lower surface ***204*** of the implant ***200.*** The groove can form a single channel as shown in **FIG. 2N1****,** or can have more than one channel as shown in **FIG. 2N2****.** In either event, the cross-bar then fits within the channel as shown in **FIGS. 2N1-N2****.** The cross-bar members ***220*** can form a solid or hollow tube or pipe structure as shown in **FIG. 2P****.** Where two, or more, tubes ***220*** communicate to provide translation, a groove ***221*** can be provided along the surface of one or both cross-members to interlock the tubes into a cross-bar member further stabilizing the motion of the cross-bar relative to the implant ***200.*** As will be appreciated by those of skill in the art, the cross-bar member ***220*** can be formed integrally with the implant without departing from the scope of the invention.

As shown in **FIGS. 2Q-R****,** it is anticipated that the surface of the tibial plateau will be prepared by forming channels thereon to receive the cross-bar members. Thus facilitating the ability of the implant to seat securely within the joint while still providing movement about an axis when the knee joint is in motion.

**FIG. 2S(1-9)** illustrate an alternate embodiment of implant ***200.*** As illustrated in **FIG. 2S** the edges are beveled to relax a sharp corner. **FIG. 2S(1)** illustrates an implant having a single fillet or bevel ***230.*** The fillet is placed on the implant anterior to the posterior portion of the tibial spine. As shown in **FIG. 2S(2)** two fillets ***230, 231*** are provided and used for the posterior chamfer. In **FIG. 2S(3)** a third fillet ***234*** is provided to create two cut surfaces for the posterior chamfer.

Turning now to **FIG. 2S(4)** a tangent of the implant is deselected, leaving three posterior curves. **FIG. 2S(5)** shows the result of tangent propagation. **FIG. 2S(6)** illustrates the effect on the design when the bottom curve is selected without tangent propagation. The result of tangent propagation and selection is shown in **FIG. 2S(7)****.** As can be seen in **FIG. 2S(8-9)** the resulting corner has a softer edge but sacrifices less than 0.5 mm of joint space. As will be appreciated by those of skill in the art, additional cutting planes can be added without departing from the scope of the invention.

**FIG. 2T** illustrates an alternate embodiment of an implant ***200*** wherein the surface of the tibial plateau ***250*** is altered to accommodate the implant. As illustrated in **FIG. 2T(1-2)** the tibial plateau can be altered for only half of the joint surface ***251*** or for the full surface ***252.*** As illustrate in **FIG. 2T(3-4)** the posterior-anterior surface can be flat ***260*** or graded ***262.*** Grading can be either positive or negative relative to the anterior surface. Grading can also be used with respect to the implants of **FIG. 2T** where the grading either lies within a plane or a body or is angled relative to a plane of the body. Additionally, attachment mechanisms can be provided to anchor the implant to the altered surface. As shown in **FIG. 2T(5-7)** keels ***264*** can be provided. The keels ***264*** can either sit within a plane, e.g. sagittal or coronal plane, or not sit within a plane (as shown in **FIG. 2T(7)). FIG. 2T(8)** illustrates an implant which covers the entire tibial plateau. The upper surface of these implants are designed to conform to the projected shape of the joint as determined under the steps described with respect to **FIG. 1****,** while the lower surface is designed to be flat, or substantially flat to correspond to the modified surface of the joint.

Turning now to **FIGS. 3A-I** an implant suitable for providing an opposing joint surface to the implant of **FIG. 2A** is shown. This implant corrects a defect on an inferior surface of the femur ***1024*** (*e.g.,* the condyle of the femur that mates with the tibial plateau) and can be used alone, *i.e.,* on the femur ***1024,*** or in combination with another joint repair device. Formation of the surfaces of the devices can be achieved using the techniques described above with respect to the implant of **FIG. 2****.**

**FIG. 3A** shows a perspective view of an implant ***300*** having a curved mating surface ***302*** and convex joint abutting surface ***304.*** The joint abutting surface ***304*** need not form an anatomic or near anatomic fit with the femur in view of the anchors ***306*** provided to facilitate connection of the implant to the bone. In this instance, the anchors ***306*** are shown as pegs having notched heads. The notches facilitate the anchoring process within the bone. However, pegs without notches can be used as well as pegs with other configurations that facilitate the anchoring process or cruciate stems. Pegs and other portions of the implant can be porous coated. The implant can be inserted without bone cement or with use of bone cement. The implant can be designed to abut the subchondral bone, *i.e.* it can substantially follow the contour of the subchondral bone. This has the advantage that no bone needs to be removed other than for the placement of the peg holes thereby significantly preserving bone stock.

The anchors ***306*** could take on a variety of other forms without departing from the scope of the invention while still accomplishing the same objective of providing increased stability of the implant ***300*** in the joint. These forms include, but are not limited to, pins, bulbs, balls, teeth, etc. Additionally, one or more joint anchors ***306*** can be provided as desired. As illustrated in **FIG. 3****,** three pins are used to anchor the implant *300.* However, more or fewer joint anchors, cruciate stems, or pins, can be used without departing from the scope of the invention.

**FIG. 3B** shows a slightly perspective superior view of the bone mating surface ***304*** further illustrating the use of three anchors ***306*** to anchor the implant to the bone. Each anchor ***306*** has a stem ***310*** with a head ***312*** on top. As shown in **FIG. 3C****,** the stem ***310*** has parallel walls such that it forms a tube or cylinder that extends from the bone mating surface ***304.*** A section of the stem forms a narrowed neck ***314*** proximal to the head ***312.*** As will be appreciated by those of skill in the art, the walls need not be parallel, but rather can be sloped to be shaped like a cone. Additionally, the neck ***314*** need not be present, nor the head ***312.*** As discussed above, other configurations suitable for anchoring can be used without departing from the scope of the invention.

Turning now to **FIG. 3D****,** a view of the tibial plateau mating surface ***302*** of the implant ***300*** is illustrated. As is apparent from this view, the surface is curved such that it is convex or substantially convex in order to mate with the concave surface of the plateau. **FIG. 3E** illustrates the upper surface ***304*** of the implant ***300*** further illustrating the use of three pegs ***306*** for anchoring the implant ***300*** to the bone. As illustrated, the three pegs ***306*** are positioned to form a triangle. However, as will be appreciated by those of skill in the art, one or more pegs can be used, and the orientation of the pegs ***306*** to one another can be as shown, or any other suitable orientation that enables the desired anchoring. **FIG. 3F** illustrated a cross section of the implant ***300*** taken along the lines F-F shown in **FIG. 3E****.** Typically the pegs are oriented on the surface of the implant so that the peg is perpendicular to the femoral condyle, which may not result in the peg being perpendicular to the surface of the implant.

**FIG. 3G** illustrates the axial view of the femur ***1000*** having a lateral condyle ***1002*** and a medial condyle ***1004.*** The intercondylar fossa is also shown ***1006*** along with the lateral epicondyle ***1008*** and medial epicondyle ***1010.*** Also shown is the patellar surface of the femur ***1012.*** The implant ***300*** illustrated in **FIG. 3A****,** is illustrated covering a portion of the lateral condyle. The pegs ***306*** are also shown that facilitate anchoring the implant ***300*** to the condyle.

**FIG. 3H** illustrates a knee joint ***1020*** from an anterior perspective. The implant ***300*** is implanted over a condyle. As shown in **FIG. 3I** the implant ***300*** is positioned such that it communicates with an implant ***200*** designed to correct a defect in the tibial plateau, such as those shown in **FIGS. 2****.**

**FIGS. 3J-K** illustrate an implant ***300*** for placement on a condyle. In this embodiment, at least one flat surface or chamfer cut ***360*** is provided to mate with a cut made on the surface of the condyle in preparing the joint. The flat surface ***360*** typically does not encompass the entire proximal surface ***304*** of the implant ***300.***

**FIG. 4A** illustrates the design of a typical total knee arthroplasty ("TKA") primary knee ***499.*** Posterior cuts ***498,*** anterior cuts ***497*** and distal cuts *496* are provided as well as chamfer cuts ***495.***

**FIGS. 4B** and **4C** illustrate another implant ***400.*** As shown in **FIG. 4B****,** the implant ***400*** is configured such that it covers both the lateral and medial femoral condyle along with the patellar surface of the femur ***1012.*** The implant ***400*** has a lateral condyle component ***410*** and a medial condyle component ***420*** and a bridge ***430*** that connects the lateral condyle component ***410*** to the medial condyle component ***420*** while covering at least a portion of the patellar surface of the femur ***1012.*** The implant ***400*** can optionally oppose one or more implants, such as those shown in **FIG. 2****,** if desired. **FIG. 4c** is a side view of the implant of **FIG. 4B****.** As shown in **FIG. 4c****,** the superior surface ***402*** of the implant ***400*** is curved to correspond to the curvature of the femoral condyles. The curvature can be configured such that it corresponds to the actual curvature of one or both of the existing femoral condyles, or to the curvature of one or both of the femoral condyles after resurfacing of the joint. One or more pegs ***430*** can be provided to assist in anchoring the implant to the bone. As will be appreciated by those of skill in the art, the implant can be configured such that the superior surface contacting a first condyle is configured to male with the existing condyle while a surface contacting a second condyle has one or more flat surfaces to mate with a condyle surface that has been modified.

**FIG. 4D** illustrates a top view of the implant ***400*** shown in **FIG. 4B****.** As is should be appreciated from this view, the inferior surface ***404*** of the implant ***400*** is configured to conform to the shape of the femoral condyles, e.g. the shape healthy femoral condyles would present to the tibial surface in a non-damaged joint.

**FIGS. 4E** and **F** illustrate perspective views of the implant from the inferior surface (*i.e.,* tibial plateau mating surface).

**FIG. 4G** illustrates the axial view of the femur ***1000*** having a lateral condyle ***1002*** and a medial condyle ***1004.*** The intercondylar fossa is also shown ***1006*** along with the lateral epicondyle ***1008.*** The implant ***400*** illustrated in **FIG. 4B****,** is illustrated covering both condyles and the patellar surface of the femur ***1012.*** The pegs ***430*** are also shown that facilitate anchoring the implant ***400*** to the condyle.

**FIG. 4H** illustrates a knee joint ***1050*** from an anterior perspective. The implant ***400*** is implanted over both condyles. As shown in **FIG. 4I** the implant ***400*** is positioned such that it communicates with an implant ***200*** designed to correct a defect in the tibial plateau, such as those shown in **FIGS. 2****.**

As will be appreciated by those of skill in the art, the implant ***400*** can be manufactured from a material that has memory such that the implant can be configured to snap-fit over the condyle. Alternatively, it can be shaped such that it conforms to the surface without the need of a snap-fit.

**FIGS. 5A** and **5B** illustrate yet another implant ***500*** suitable for repairing a damaged condyle. As shown in **FIG. 5A****,** the implant ***500*** is configured such that it covers only one of the lateral or medial femoral condyles ***510.*** The implant differs from the implant of **FIG. 3** in that the implant ***500*** also covers at least a portion of the patellar surface of the femur ***512.***

Similar to the implant of **FIG. 4****,** the implant can optionally oppose one or more implants or opposing joint surfaces, such as those shown in **FIG. 2****,** and can be combined with other implants, such as the implants of **FIG. 3****.** **FIG. 5c** is a perspective side view of the implant of **FIG. 5A****.** As shown in **FIG.5C****,** the superior surface ***502*** of the implant ***500*** is curved to correspond to the curvature of the femoral condyle that it mates with and the portion of the patellar surface of the femur that it covers. One or more pegs ***530*** can be provided to assist in anchoring the implant to the bone. Additionally, an angled surface ***503*** can be provided on an interior surface ***502*** of the condyle component that conforms to an optionally provided cut made on the surface of the joint surface with which the implant mates.

**FIG. 5D** illustrates a perspective top view of the implant ***500*** shown in **FIG. 5A****.** As is should be appreciated from this view, the inferior surface ***504*** of the implant ***500*** is configured to conform to the projected shape of the femoral condyles, *e.g.* the shape healthy femoral condyles would present to the tibial surface in a non-damaged joint.

**FIG. 5E** is a view of the implant ***500*** showing a hatched three point loading support area which extends from a top portion ***513*** to a line (plane 17) and from a line (plane 18) to a bottom portion ***515.*** Also illustrated are the pegs ***530*** extending from the superior surface. **FIG. 5F** illustrates the superior surface of the implant ***500*** with the pegs ***530*** extending from the superior surface. **FIG. 5F** also illustrates the hatched cantilever loading support area, which extends from the line (plane 18) to the top portion ***513*** of the implant. The loading forces and directions for each support condition are based on physiological load encounters. Table 1 shows the Physiological Loadings taken from a study by Seth Greenwald

**Table 1**

| **Physiological Loadings¹** | | | |
|---|---|---|---|
| **Set-up** | **"1"** | **"2"** | **"3"** |
| **Flexion Angle (degree)** | 0° | 60° | 90° |
| **Normal Force N (lbs.)** | 2,900 (652) | 3,263 (733.5) | 3,625 (815) |
| **Normal Force Case** | Walking (4.0 x BW^{¥}) | Stair Descent (4.5 x BW^{¥}) | Stair Ascent (5.0 x BW^{¥}) |

| | | | |
|---|---|---|---|
| ^{¥}Body Weight (BW) taken as a 60 year old male, with 173 cm height for an average body weight of 74 kg (163 lbs). ¹"Tibial Plateau Surface Stress in TKA: A Factor Influencing Polymer Failure Series III - Posterior Stabilized Designs;" Paul D. Postak, B.Sc., Christine S. Heim, B.Sc., A. Seth Greenwald, D. Phil.; Orthopaedic Research Laboratories, The Mt. Sinai Medical Center, Cleveland, Ohio. Presented at the 62nd Annual AAOS Meeting, 1995. | | | |

Using the implant ***500*** described in this application, the three point loading will occur from set-up 1 (2900 N). To replicate a worst case loading scenario, a 75/25 load distribution (75% of 2900 N = 2175 N) will be used. The loading will be concentrated on a 6mm diameter circular area located directly below and normal to the ped on the bearing surface.

Turning to the cantilever loading shown in **FIG. 5F****,** the loading will occur from set-up 3, or 90°, at a 75/25 load distribution (75% of 3625 N = 2719 N). As with the above example, the loading will be concentrated on a 6 mm diameter circular area located at the center of the posterior-most portion of the medial condyle normal to the flat cut surface of the posterior condyle.

**FIGS. 5G** and H illustrate alternate embodiments of the implant ***500*** having a rail design that provides one or more rails ***521*** along medial and/or lateral sides of the implant ***500.*** The rail ***521*** can be positioned so that it extends along a portion of the medial ***517*** and/or lateral ***519*** sides before communicating with the angled surface ***503.*** As will be appreciate, a single side rail ***521*** can be provided without departing from the scope of the invention.

**FIG. 5I** illustrates another embodiment of an implant ***500*** having a keel design. A keel ***523*** (or centrally formed rail) is provided on the superior surface of the implant. In this embodiment, the keel ***523*** is located on the surface of the implant, but not at the sides. As will be appreciated, the keel can be centered, as shown, substantially centered, or located off-center. An angled surface ***503*** can be provided to communicate with a modified joint surface. Alternatively, where the joint surface is worn or modified, the cut ***503*** could be configured to mate with the worn or modified surface.

**FIG. 5J** illustrates the axial view of the femur ***1000*** having a lateral condyle ***1002*** and a medial condyle ***1004.*** The intercondylar fossa is also shown ***1006*** along with the lateral epicondyle ***1008*** and the medial epicondyle ***1010.*** The patellar surface of the femur ***1012*** is also illustrated. The implant ***500,*** illustrated in **FIG. 5A****,** is shown covering the lateral condyle and a portion of the patellar surface of the femur ***1012.*** The pegs ***530*** are also shown that facilitate anchoring the implant ***500*** to the condyle and patellar surface.

**FIG. 5K** illustrates a knee joint *1020* from an anterior perspective. The implant ***500*** is implanted over the lateral condyle. **FIG. 5L** illustrates a knee joint ***1020*** with the implant ***500*** covering the medial condyle *1004.* As illustrated in **FIGS. 5K** and **L** the shape of the implant ***500*** corresponding to the patella surface can take on a variety of curvatures without departing from the scope of the invention.

Turning now to **FIG. 5M** and **N** the implant ***500*** is positioned such that it communicates with an implant 200 designed to correct a defect in the tibial plateau, such as those shown in **FIGS. 2****.**

In another embodiment of the invention, the implant ***500*** can have a superior surface ***502*** which substantially conforms to the surface of the condyle but which has at one flat portion corresponding to an oblique cut on the bone as shown in **FIG. 5O****.**

Turning now to **FIG. 5P-Q** an implant ***500*** is shown from a side view with a 7° difference between the anterior and posterior cuts.

**FIG. 5R-S** illustrate an implant ***500*** having a contoured surface ***560*** for mating with the joint surface and an anterior cut ***561*** and a posterior cut ***562.*** **FIG. 5s** shows the same implant ***500*** from a slightly different angle. **FIG. 5T** illustrates another implant ***500*** having a contoured surface ***560*** for mating with the joint surface and posterior cut ***562,*** a distal cut ***563,*** and a chamfer cut ***564.*** In this embodiment no anterior cut is provided. **FIG. 5U** illustrates the implant ***500*** of **FIG. 5T** from a side perspective. The cuts are typically less than the cut required for a TKA, i.e., typically less than 10mm. The design of the cuts for this implant allow for a revision surgery to the knee, if required, at a later date.

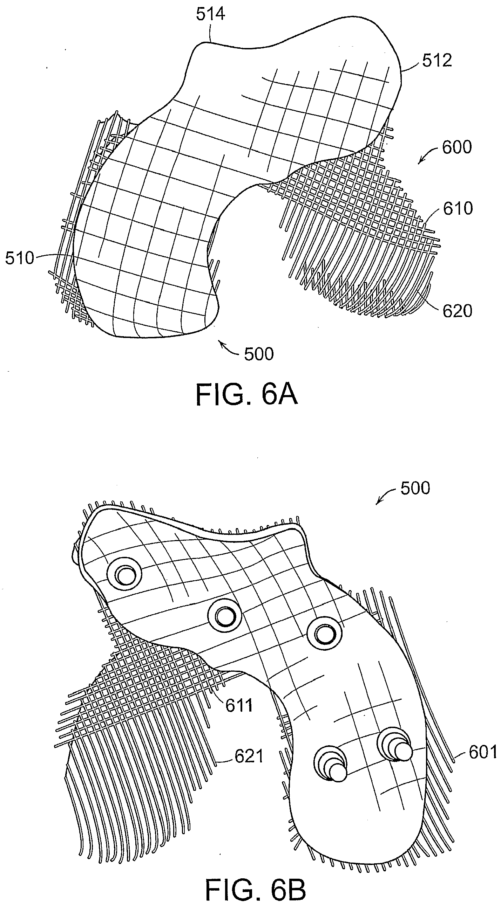

**FIGS. 6A-G** illustrate the implant ***500*** of **FIG. 5** with a graphical representation of the cross-sections ***610, 620*** from which a surface shape of the implant is derived. **FIG. 6A** illustrates a top view of the implant ***500*** sitting on top of the extracted surface shape ***600.*** This view of the implant ***500*** illustrates a notch ***514*** associated with the bridge section of the implant ***512*** which covers the patellar surface of the femur (or the trochlea region) to provide a mating surface that approximates the cartilage surface. As will be appreciated by those of skill in the art, the shape of an implant designed for the medial condyle would not necessarily be a mirror image of the implant designed for the lateral condyle because of differences in anatomy. Thus, for example, the notch ***514*** would not be present in an implant designed for the medial condyle and the patellar surface of the femur. Therefore, the implant can be designed to include all or part of the troclea region or to exclude it entirely.

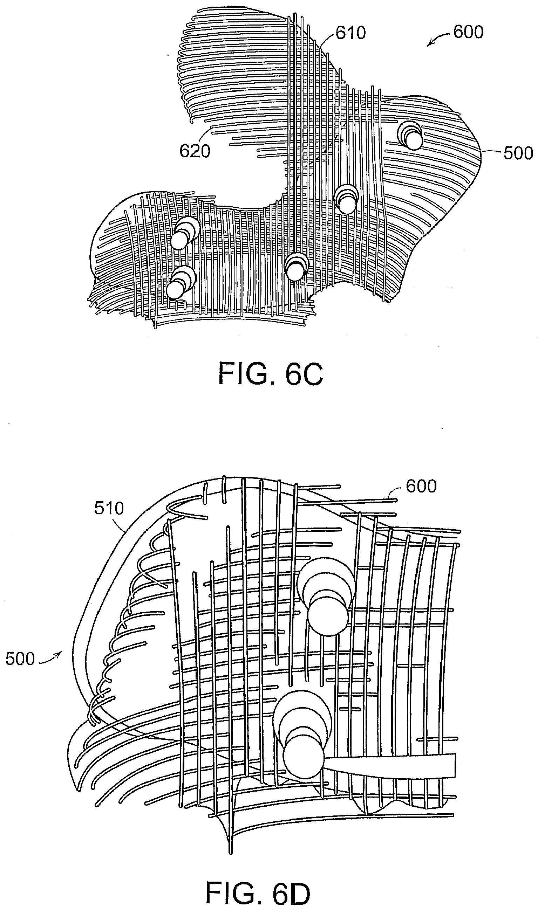

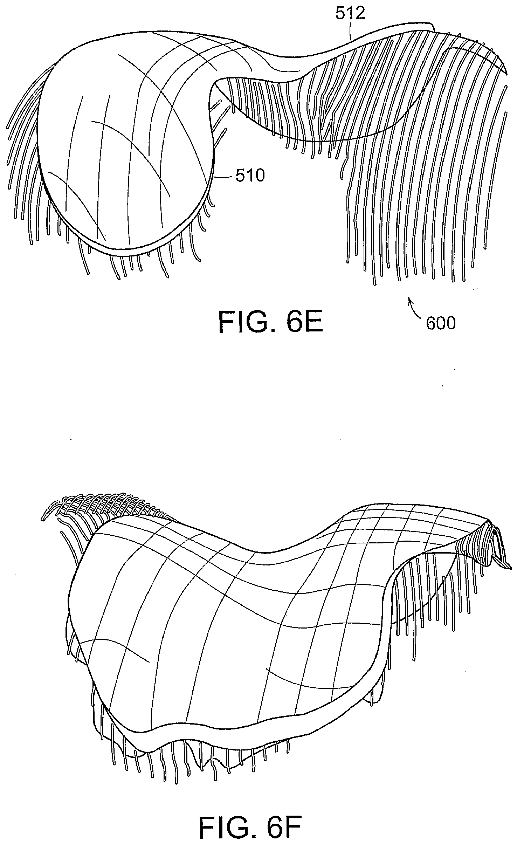

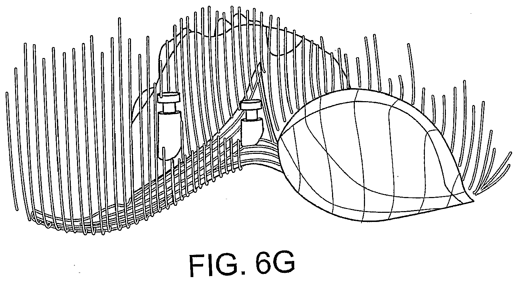

**FIG. 6B** illustrates a bottom view of the implant ***500*** layered over another derived surface shape ***601.*** **FIG. 6C** is a bottom view showing the implant ***500*** extending through the extracted surface shape ***600*** shown in **FIG. 6A****.** **FIG. 6D** is a close-up view of the **FIG. 6c** showing the condylar wing of the implant covering the extracted surface ***600.*** **FIG. 6E** illustrates a top posterior view of the implant ***500*** positioned over the graphical representation of the surface shape ***600.*** **FIG. 6F** is an anterior view and **FIG. 6G** is a bottom-posterior view.

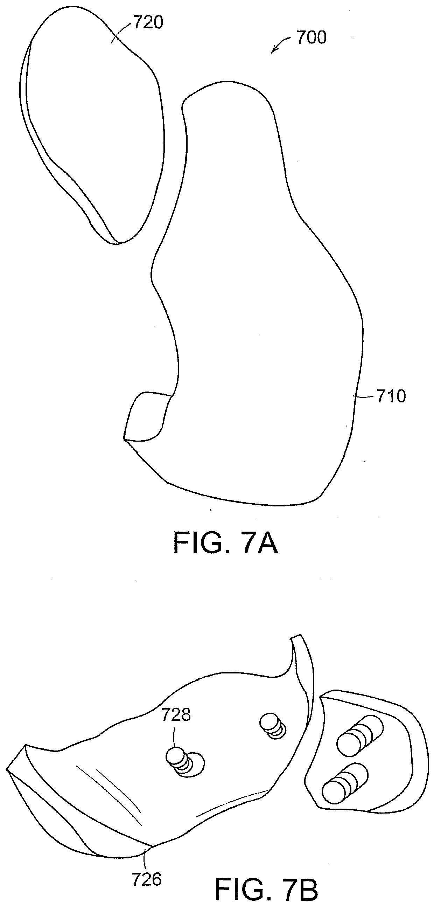

**FIG. 7A-C** illustrate an implant ***700*** for correcting a joint similar to the implant ***500*** above. However, implant ***700*** consists of two components. The first component ***710*** engages a condyle of the femur, either medial or lateral depending on the design. The second component ***720*** engages the patellar surface of the femur. As discussed with the previous embodiments, the surfaces of the implant ***700*** can be configured such that the distal surface ***722*** (e.g., the surface that faces the tibial plateau) is shaped based on a projection of the natural shape of the femur compensating the design for valgus or varus deformities and/or flattening of the surface of the femur. Alternatively, the distal surface can be shaped based on the shape of the tibial plateau to provide a surface designed to optimally mate with the tibial plateau. The proximal surface ***724*** (e.g., the surface that engages the femoral condyle) can be configured such that it mirrors the surface of the femur in either its damaged condition or its modified condition. Likewise, the proximal surface can have one or more flattened sections ***726*** that form, e.g., chamfer cuts. Additionally the surface can include mechanisms facilitating attachment **728** to the femur, such as keels, teeth, cruciate stems, and the like. The medial facing portion of the condyle implant has a tapered surface ***730*** while the lateral facing portion of the patellar component also has a tapered surface such that each component presents tapered surfaces ***730*** to the other component.

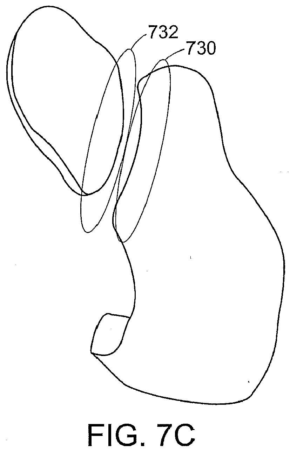

By dividing the surfaces of the medial and lateral compartments into independent articulating surfaces, as shown in **FIG. 7****,** the implant provides improved fit of the conformal surfaces to the subchondral bone. Additionally, the lateral-anterior portion of the femur is shielded from stress which could cause bone loss. Also, the smaller size of each component of the implant, enables the implant to be placed within the joint using a smaller incision. Finally, the wear of the patellar component is improved.

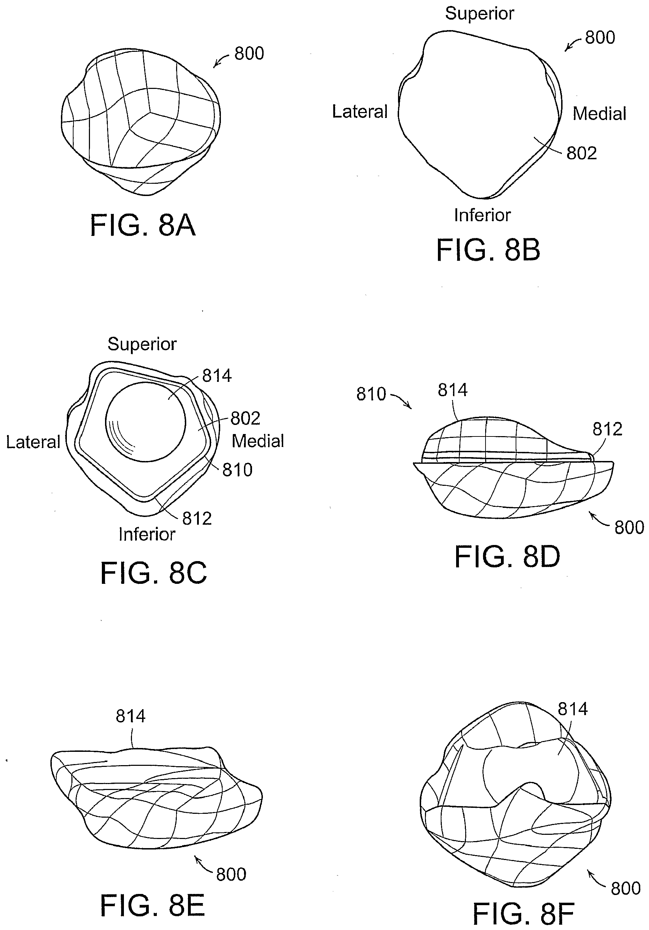

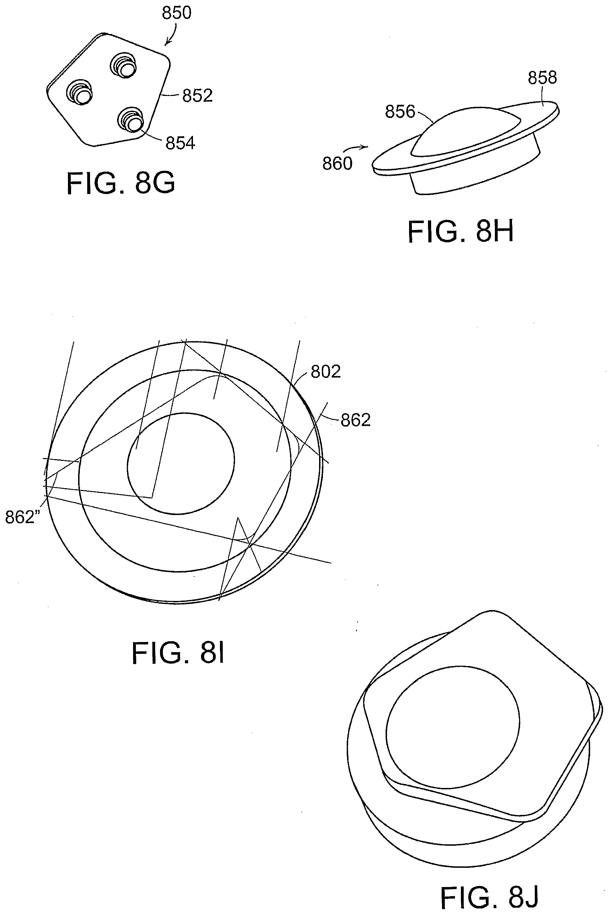

**FIGS. 8A-F** illustrate a patella ***00*** with an implants ***810.*** The implant ***810*** can have one or more pegs, cruciate stems, or other anchoring mechanisms, if desired. As will be appreciated by those of skill in the art, other designs can be arrived at using the teachings of this disclosure without departing from the scope of the invention. **FIG. 8A** illustrates a perspective view of an intact patella ***800.*** **FIG. 8B** illustrates the patella ***800*** wherein one surface of the patella ***800*** has been cut for form a smooth surface ***802*** to mate with an implant. **FIG. 8c** illustrates the patella ***800*** with an implant ***810*** positioned on the smooth surface ***802.*** The implant ***810*** has a plate structure ***812*** that abuts the smooth surface of the patella ***802*** and a dome ***814*** positioned on the plate ***812*** so that the dome is positioned in situ such that it will match the location of the patellar ridge. The implant ***810*** can be configured such that the edge of the plate is offset 1 mm from the actual edge of the patella, as illustrated. As will be appreciated by those of skill in the art, the plate ***812*** and dome ***814*** can be formed as a single unit or formed from multiple components. **FIG. 8D** is a side view of the implant ***810*** positioned on the patella ***800.*** As shown, the dome is positioned on the implant such that it is off-center. Optimal positioning of the dome will be determined by the position of the patellar ridge.

Turning now to **FIGS. 8E-F****,** the implant ***810*** is shown superimposed on the unaltered patella ***800*** in order to illustrate that the position of the dome ***814*** of the implant corresponds to the location of the patellar ridge.

**FIGS. 8G-J** illustrate an alternative design for the patellar implant. **FIG. 8G** illustrates the implant ***850*** in its beginning stages as a blank with a flat inferior surface ***852*** having pegs ***854*** extending there from for anchoring to the patella. The articular or superior surface ***860*** has a rounded dome ***856,*** and a round plate section ***858*** that can be machined to match the bone cut. The articular surface ***860*** takes on the appearance of a "hat" or sombrero, having a dome with a rim. The center of the dome ***856*** is also the center of the bearing surface. The rim ***858*** is cut to conform to the needs of the particular patient. **FIG. 8J** illustrates an implant which has been formed from the blank shown in **FIGS. 8G-I. FIG. 8I** shows a plurality of possible cut lines ***862, 862'*** for purposes of illustration.

**FIGS. 9A-C** illustrate a lateral view of a knee ***1020*** having a combination of the implants of implanted thereof. In **FIG. 9A****,** an implant covering the condyle ***900,*** is illustrated. Suitable implants can be, for example, those shown in **FIGS. 3-8****,** as will be appreciated the portion of the condyle covered anterior to posterior can include the entire weight bearing surface, a portion thereof, or a surface greater than the weight bearing surface. Thus, for example, the implant can be configured to terminate prior to the sulcus terminalis or after the sulcus terminalis (e.g., the groove on the femur that coincides with the area where load bearing on the joint surface stops). As shown in **FIGS. 9A-B****,** a patellar implant ***900*** can also be provided. **FIG. 9C** illustrates a knee having a condyle implant ***900,*** a patellar implant ***800*** and an implant for the tibial plateau ***200.***

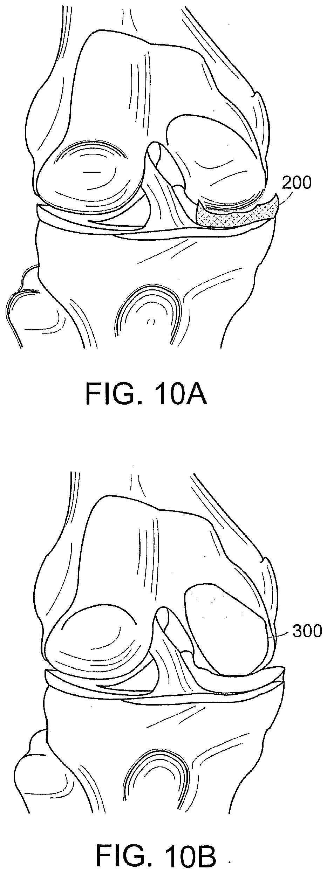

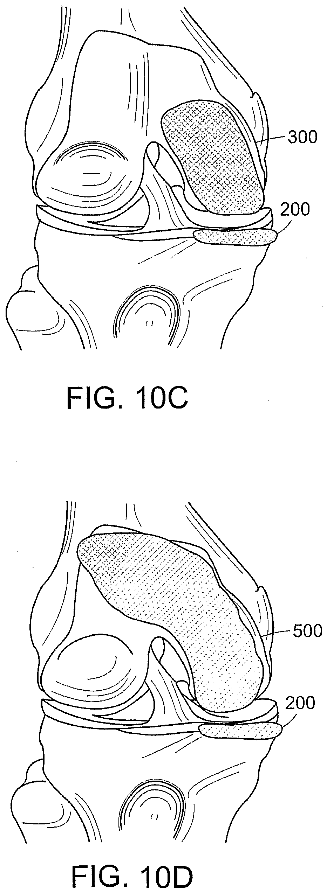

**FIGS. 10A-D** provide an alternate view of the coronal plane of a knee joint with one or more implants described above implanted therein. **FIG. 10A** illustrates a knee having a tibial implant ***200*** placed therein. **FIG. 10B** illustrates a knee with a condyle implant ***300*** placed therein. As described above, a plurality of the implants taught herein can be provided within a joint in order to restore joint movement. **FIG. 10C** illustrates a knee joint having two implants therein. First, a tibial implant ***200*** is provided on the tibial plateau and a second implant ***300*** is provided on the facing condyle. As will be appreciated by those of skill in the art. The implants can be installed such that the implants present each other mating surfaces (as illustrated), or not. For example, where the tibial implant ***200*** is placed in the medial compartment of the knee and the condyle implant ***300*** is placed in the lateral compartment. Other combinations will be appreciated by those of skill in the art. Turning now to **FIG. 10D****,** a tibial implant ***200*** is provided along with a bicompartmental condyle implant ***500.*** As discussed above, these implants can be associated with the same compartment of the knee joint, but need not be.

The arthroplasty system can be designed to reflect aspects of the tibial shape, femoral shape and/or patellar shape. Tibial shape and femoral shape can include cartilage, bone or both. Moreover, the shape of the implant can also include portions or all components of other articular structures such as the menisci. The menisci are compressible, in particular during gait or loading. For this reason, the implant can be designed to incorporate aspects of the meniscal shape accounting for compression of the menisci during loading or physical activities. For example, the undersurface ***204*** of the implant ***200*** can be designed to match the shape of the tibial plateau including cartilage or bone or both. The superior surface ***202*** of the implant ***200*** can be a composite of the articular surface of the tibia (in particular in areas that are not covered by menisci) and the meniscus. Thus, the outer aspects of the device can be a reflection of meniscal height. Accounting for compression, this can be, for example, 20%, 40%, 60% or 80% of uncompressed meniscal height.

Similarly the superior surface ***304*** of the implant ***300*** can be designed to match the shape of the femoral condyle including cartilage or bone or both. The inferior surface ***302*** of the implant ***300*** can be a composite of the surface of the tibial plateau (in particular in areas that are not covered by menisci) and the meniscus. Thus, at least a portion of the outer aspects of the device can be a reflection of meniscal height. Accounting for compression, this can be, for example, 20%, 40%, 60% or 80% of uncompressed meniscal height. These same properties can be applied to the implants shown in **FIGS. 4-8****,** as well.

In some embodiments, the outer aspect of the device reflecting the meniscal shape can be made of another, preferably compressible material. If a compressible material is selected it is preferably designed to substantially match the compressibility and biomechanical behavior of the meniscus. The entire device can be made of such a material or non-metallic materials in general.

The height and shape of the menisci for any joint surface to be repaired can be measured directly on an imaging test. If portions, or all, of the meniscus are torn, the meniscal height and shape can be derived from measurements of a contralateral joint or using measurements of other articular structures that can provide an estimate on meniscal dimensions.

In another embodiment, the superior face of the implants ***300, 400*** or ***500*** can be shaped according to the femur. The shape can preferably be derived from the movement patterns of the femur relative to the tibial plateau thereby accounting for variations in femoral shape and tibiofemoral contact area as the femoral condyle flexes, extends, rotates, translates and glides on the tibia and menisci.

The movement patterns can be measured using any current or future test know in the art such as fluoroscopy, MRI, gait analysis and combinations thereof.

In various embodiments, a joint implant may include two or more components that are slideably engageable forming a mobile bearing. The mobile bearing can help provide more unconstrained or more physiologic motion in the joint, for example, knee motion of the femur relative to the tibia.

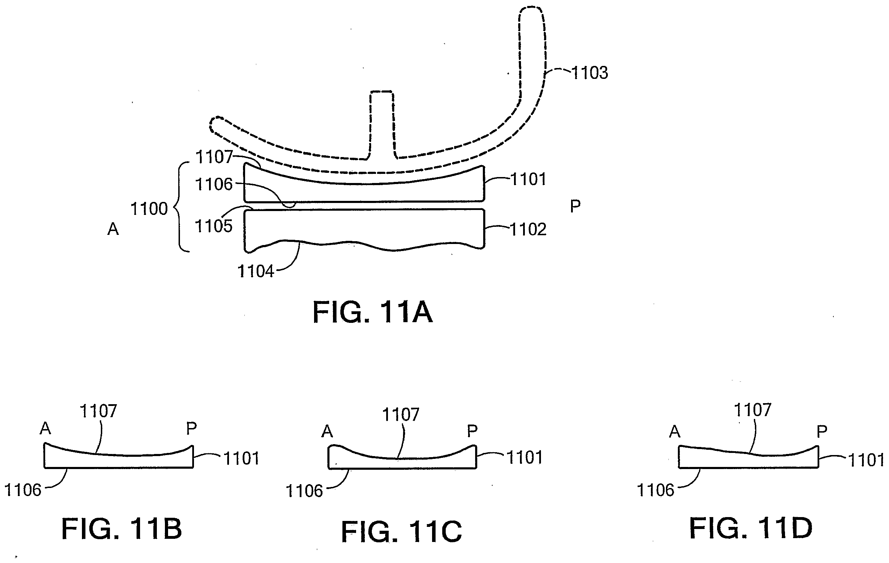

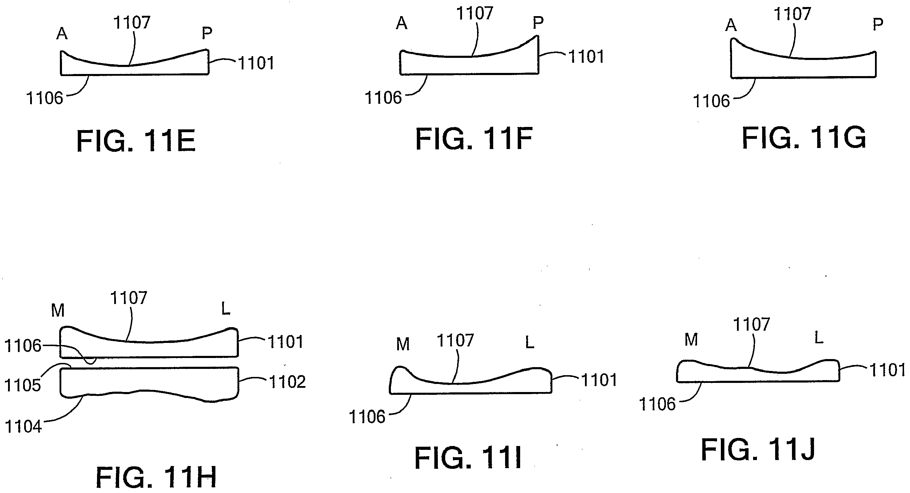

**FIG. 11A** shows a joint implant **1100** that includes a mobile bearing, in accordance with one embodiment of the invention, The implant **1100** includes a first component **1102** and a bearing component **1101.** The first component **1102** includes a bone-facing surface **1104** for engaging bone or cartilage of a joint, and an external surface **1105.** The bearing component **1101** includes a first surface **1106** for slidingly engaging the external surface of the first component **1101,** and a second surface **1107** for articulation with a second component **1103** and/or other bone or cartilage surface.

The bone facing surface **1104** may be a mirror image of, and engage, a substantially uncut articular cartilage surface and/or a substantially uncut subchondral bone surface. The bone-facing surface may be formed using, without limitation, the above-described imaging and modeling techniques. The bone facing surface **1104** may match and conform with the existing underlying surface to achieve an anatomic or near anatomic fit, such that it replicates the natural joint anatomy. Such a non-invasive approach advantageously does not require surgical resection of the articular bone surface.

In various embodiments, the joint implant **1100** may be configured to be used, without limitation, in a hip, knee, ankle, should, elbow, wrist, or hand. For example, the joint implant **1100** may be directed at a knee, with the bone facing surface **1104** of the first component **1102** engaging a tibial articular surface, and the second surface **1107** of the bearing component **1101** articulating with a femoral component **1103.**

The bearing surface between the bearing component **1101** and the first component **1102** may be, without limitation, flat, as shown in **FIG. 11A****.** The second surface of the bearing component **1101,** which may, for example, face the femur in a knee implant (or, for example, face an implant **1103** covering a portion of the femoral condyle), may have a constant or variable radii both in anteroposterior and/or mediolateral directions as shown in **FIGS. 11B-J****,** in accordance with various embodiments of the invention. For example, the second surface **1107** of the bearing component **1101** in a knee implant may include one or more concavities and/or convexities so as to match the superior surface of the replaced mensical cartilage and/or provide for proper articulation with a femoral implant and/or articular surface.

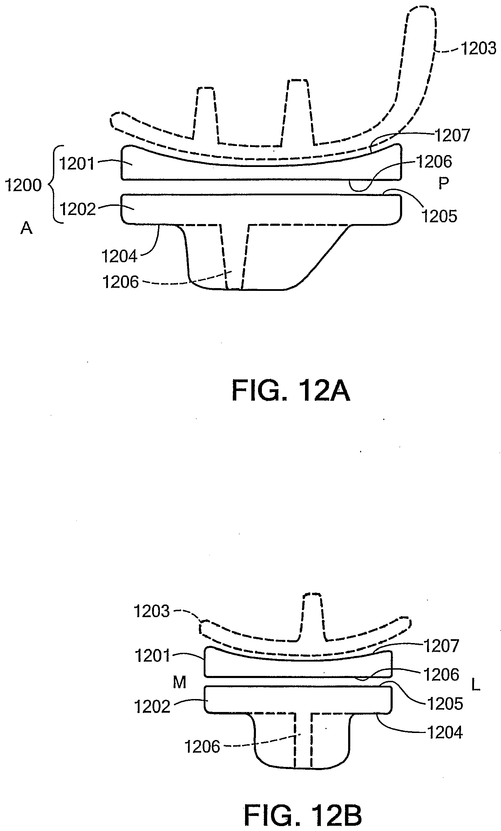

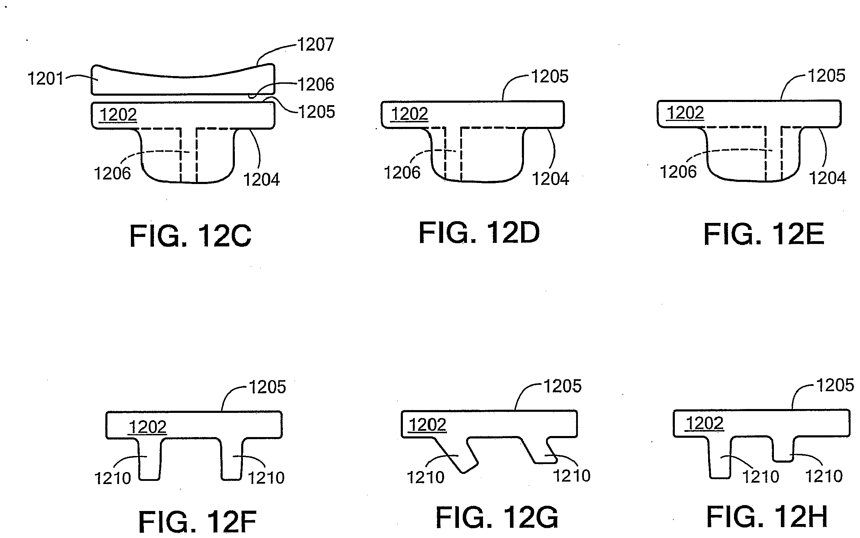

**FIGS. 12A-E** show a joint implant **1200** having a mobile bearing that can be fixedly anchored into the articular joint, such as, in a knee implant, the tibial plateau (not shown), in accordance with various embodiments of the invention. For example, one or more fins **1205** and **1206** positioned on the bone facing surface of the first component **1202** of the joint implant **1200** may be used to anchor the joint implant **1200** into the tibial plateau. The fins **1205** and **1206** may be perpendicular relative to each other or they may be oriented at an angle other than 90 degrees. A transverse fin **1206** may be located in the center of an anteroposterior fin **1205,** as shown in **FIGS. 12B****.** In other embodiments, the transverse fin **1206** may be located anterior or posterior relative to the center of the anteroposterior fin, as shown in **FIGS. 12D-E****.**

Alternatively, the joint component **1200** may be anchored via one or more pegs **1210** into, without limitation, the tibial plateau, as shown in **FIGS. 12F-H****,** in accordance with various embodiments of the invention. These pegs **1210** may be perpendicular to the articular surface, as shown in **FIG. 12F** or at an angle other than 90 degrees to the articular surface, as shown in **FIG. 12G****.** The pegs may have the same length as shown in **Figs. 12F****,** or a different length, as shown in **FIG. 2H****.** With an anterior incision, a shorter posterior peg may advantageously allow a more minimally invasive approach by reducing the size of the incision.

Any anchoring mechanism known in the art may be used.

The bone-facing surface of the first component **1202** of the joint implant 1200 may sit on top of, and conform with, the subchondral bone and/or articular cartilage without cutting the tibia, with only the anchoring mechanism protruding into the bone. Alternatively, the surgeon may cut the articular surface (e.g., the tibial plateau in a knee implant), and the implant can be seated on top of the cut. Standard techniques for cutting the articular surface known in the art may be used. Note that in various embodiments, the bone-facing surface may conform with the subchondral bone and/or articular cartilage such that the first component is sufficiently self-centering, with requiring any anchoring mechanism or cuts.

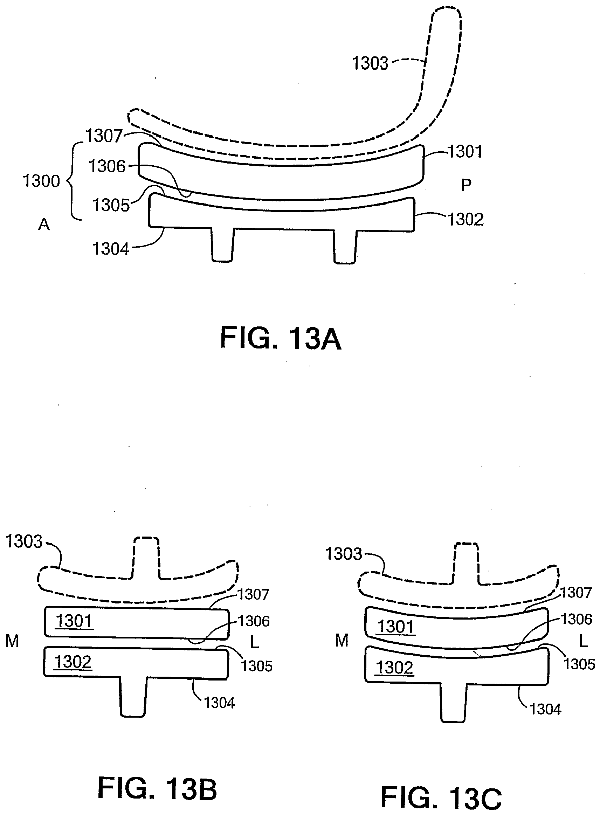

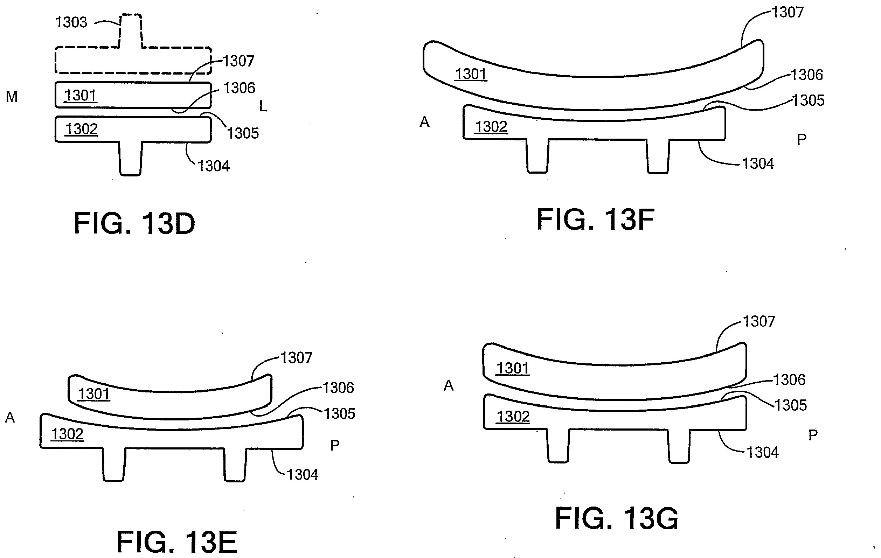

**FIG. 13A** shows a joint implant **1300** having a bearing surface between the bearing component **1301** and the first component **1302** that is curved rather than flat, in accordance with one embodiment of the invention. For example, the external surface **1305** of the first component **1302** may be concave, rising towards the bearing component **1301** upwardly on one or more sides. The second surface **1307** of the bearing component **1301** (e.g., that faces the femur in a knee implant) may have a constant or variable radii both in anteroposterior and / or mediolateral direction. A variable radius may advantageously accommodate different femoral radii during flexion and translation of the condyle.

In various embodiments, the bearing surface between the bearing component **1301** and the first component **1302** may be flat and the second surface **1307** of the bearing component **1301** (e.g., facing the femoral component in a knee implant) may also be flat, as shown in **FIG. 13B****.** In other embodiments, the bearing surface between the bearing component **1301** and first component **1302** may be curved and the second surface **1307** of the bearing component **1301** may also be curved, as shown in **FIG. 13c****.** In still other embodiments, the bearing surface between the bearing component **1301** and the first component **1302** may be flat, with the second surface **1307** of the bearing component **1301** flat, and the femoral component 1303 flat in one dimension ( preferably the coronal dimension in a knee implant), as shown in **Fig. 13D****.**

In another embodiment, the bearing component **1301** of the joint implant **1300** may be smaller in one or more dimensions than the first component **1302,** as shown in **FIG. 13E****.** In other embodiments, the bearing component **1301** may be longer in one or more dimensions than the first component **1302,** as shown in **FIG. 13F****,** or it can have substantially the same length or width or both than the first component **1302,** as shown in **FIG. 3G****.**

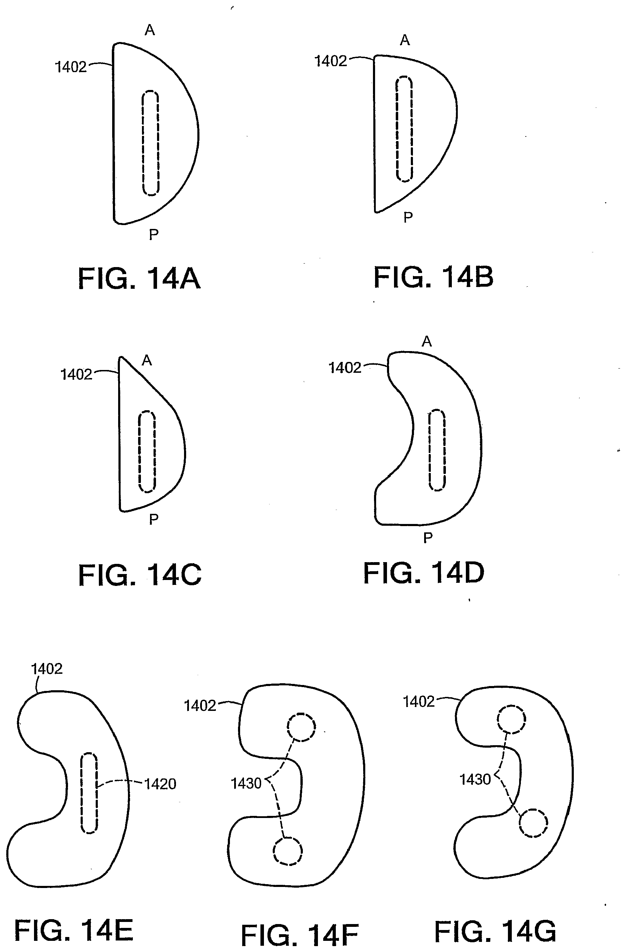

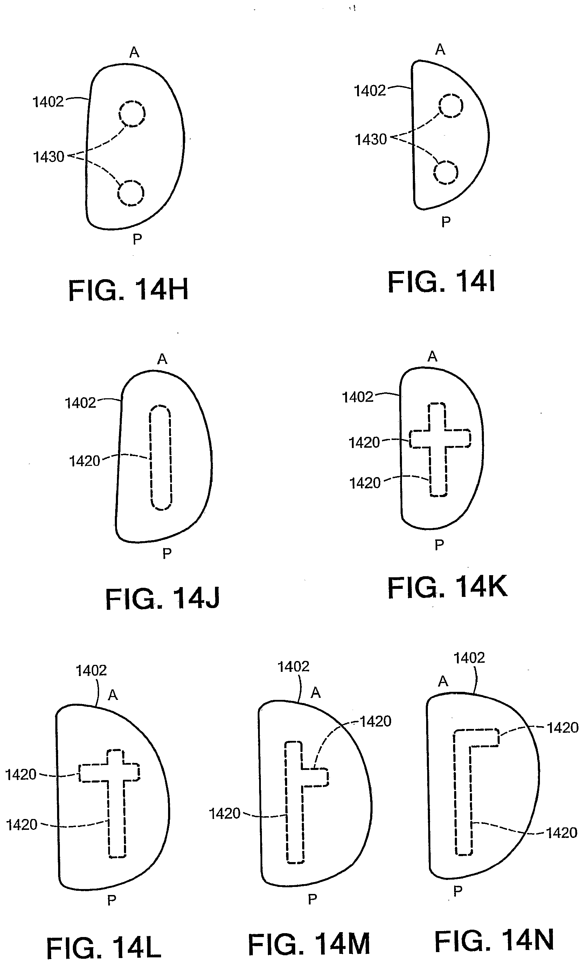

**FIGS. 14A-N** shows a top view of perimeter, keel and peg configurations for the first component **1402** of a joint implant that includes a mobile bearing, in accordance with various embodiments of the invention. **FIG. 14A** shows the first component **1402** of a joint implant, which illustratively is a tibial implant, having a perimeter with a substantially constant radius, in accordance with one embodiment of the invention. In other embodiments, the first component **1402** may have a variable radius, as shown in **FIG. 14B. FIG. 14C** shows an example of a first component **1402** with a variable radius and a substantially straight surface oriented towards the intercondylar notch area. In other embodiments, the surface oriented towards the intercondylar notch may be concave or convex in at least one portion, as shown in **FIG. 14D****.** In **FIG. 14D****,** the concavity helps avoid the tibial spines intraoperatively, with the the outer perimeter of the first component being kidney shaped, as shown in **FIGS. 14D-G. FIGS. 14E-G** demonstrate various embodiments with concave and convex tibial component perimeters and various keel **1420** (**FIG. 14E**) and peg **1430 (****FIGS. 4F-G**) positions.

**FIG. 14H** shows a first component **1402** that is substantially round in perimeter using two exemplary pegs **1430** for attachment, in accordance with one embodiment of the invention. **FIG. 14I** shows an exemplary semilunar shaped first component **1402** using two exemplary pegs **1430** for attachment. The outer perimeter of the first component **1402** may be substantially round (**FIG. 14H**) or can include more pointed aspects (**FIG. 14I**).

**Fig. 14J** shows a first component **1402** with a single fin **1420** for anchoring, in accordance with one embodiment of the invention. A first component **1402** with two perpendicularly arranged keels **1420** for anchoring is shown in **FIG. 14K****,** in accordance with another embodiment of the invention.

**Fig. 14L** shows a first component **1402** with the fin **1420** located in substantially the coronal plane moved more anteriorly relative to the fin **1420** located in substantially the sagittal plane, in accordance with one embodiment of the invention. **FIGS. 14M-N** show other examples of potential configurations of the keels **1420.**

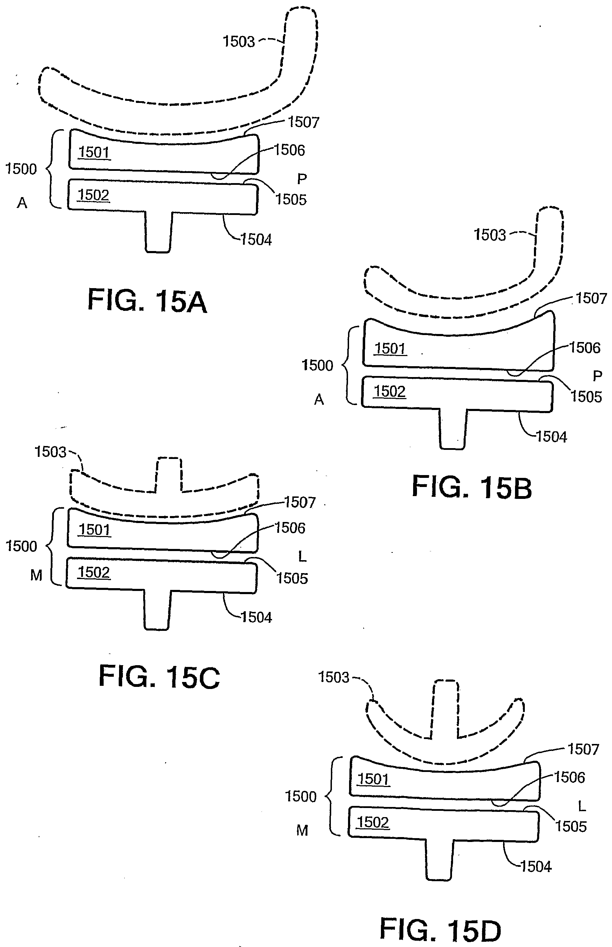

**FIG. 15A** shows a joint implant **1500** with the second surface **1507** of the bearing component **1501** having a constant radius in the sagittal plane, in accordance with one embodiment of the invention. **FIG. 15B** shows a joint implant **1500** with the second surface **1507** of the bearing component **1501** having a variable radius in the sagittal plane. **Fig. 15c** shows a joint implant **1500** with the second surface **1507** of the bearing component **1501** having a constant radius substantially matching that of, for example, the femoral condyle (or, for example, matching an implant **1503** covering a portion of the femoral condyle) in the coronal plane. **FIG. 15D** shows a joint implant **1500** with the second surface **1507** of the bearing component **1501** having a constant radius not substantially matching, for example, that of the femoral condyle (or, for example, not matching an implant **1503** covering a portion of the femoral condyle) in the coronal plane.

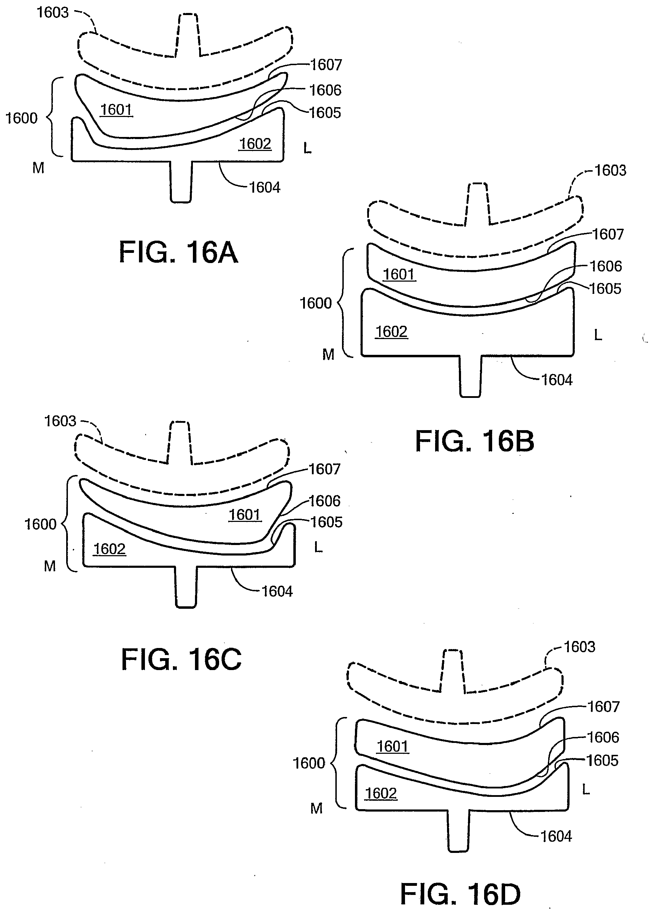

**FIG. 16A** shows a joint implant **1600** with a bearing surface between the bearing component **1601** and the first component **1602** that is asymmetrical, with varying curvature radii, in accordance with one embodiment of the invention. Radii may vary in one or more dimensions. Radii may be chosen such that the external surface **1605** of the first component **1602** and the first surface **1606** of the bearing component **1601** simulate near physiologic motion of the components, for example matching, in a knee implant, tibiofemoral rotation and translation. The bearing surface may include, without limitation, one or more convexities and/or concavities. The second surface **1607** of the bearing component **1601** facing, in a knee implant for example, the femoral condyle has, without limitation, a substantially constant radius.

**FIG. 16B** shows a joint implant **1600** with a bearing surface between the bearing component **1601** and the first component **1602** that is symmetrical, with constant radii, in accordance with one embodiment of the invention. Radii may be constant in only one dimension or more.

**FIG. 16C** shows a joint implant **1600** with a bearing surface between the bearing component **1601** and the first component **1602** that is asymmetrical, with varying radii. The second surface **1607** of the bearing component **1601** facing, in a knee implant for example, the femoral condyle has, without limitation, a substantially constant radius.

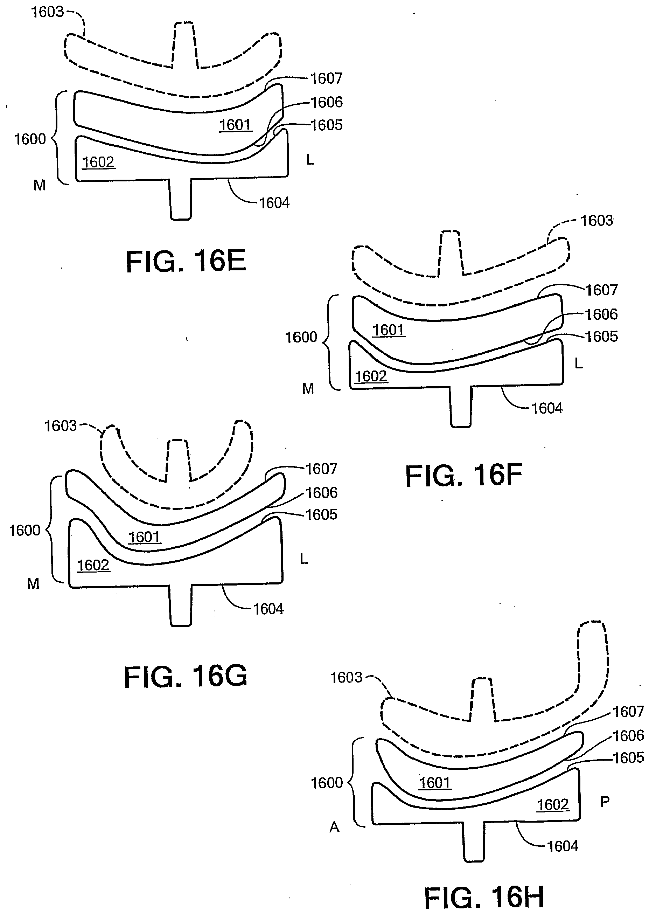

**FIGS. 16D-E** demonstrate joint implants **1600** with a bearing surface between the bearing component **1601** and the first component **1602** that is asymmetrical, with varying radii, and the second surface **1607** of the bearing component **1601** that has also varying radii, in accordance with various embodiments of the invention. In **FIG. 16D****,** the second component (e.g. the femoral component in a knee implant) **1603** has a substantially constant radius. In **FIG. 16E****,** the second component **1603** has a variable radius.

**FIG. 16F** shows a joint implant **1600** having a second surface **1607** of the bearing component **1601** with variable radii that are, however, substantially matching the radii of the second component **1603,** in accordance with one embodiment of the invention. **FIG. 16G** shows a joint implant **1600** having a second surface **1607** of the bearing component **1601** with variable radii that are, however, substantially not matching the radii of the second component **1603,** in accordance with one embodiment of the invention.

**FIG. 16H** shows a joint device **1600,** such as a tibial implant, in the sagittal plane with the bearing surface of the bearing component **1601** and the first component **1602** having variable radii in the sagittal plane, and the second surface **1607** of the bearing component **1601** facing the second component **1603** (e.g., the femoral condyle) having a substantially constant radius, in accordance with one embodiment of the invention. The smallest radii are observed anteriorly in the bearing surface.

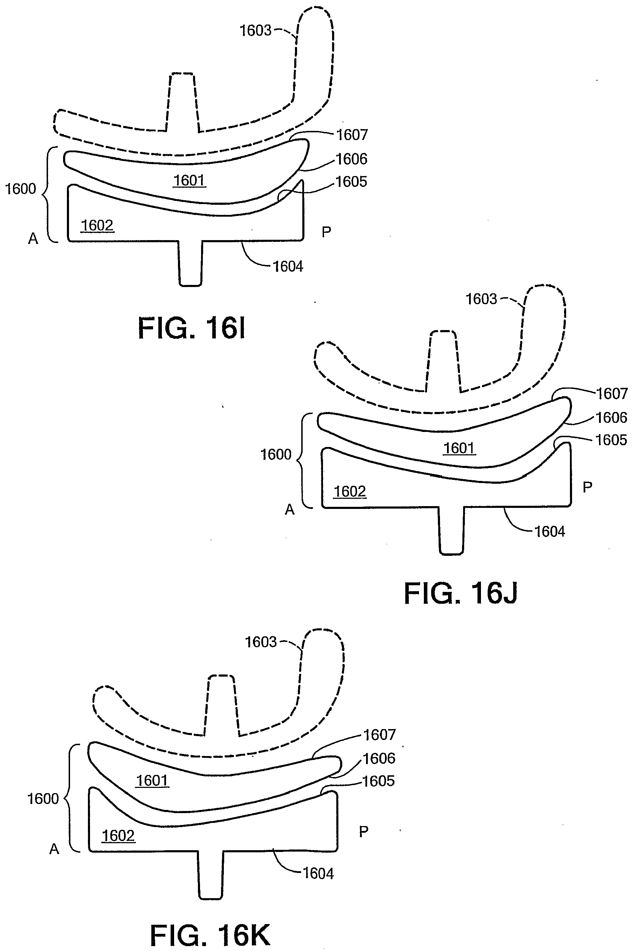

**FIG. 16I** shows a joint device **1600,** such as a tibial implant, in the sagittal plane with the bearing surface of the two components **1601** and **1602** having variable radii in the sagittal plane, and the second surface **1607** of the bearing component **1601** facing the second component **1603** (e.g., the femoral condyle) having a substantially constant radius, in accordance with one embodiment of the invention. The smallest radii are observed posteriorly in the bearing surface.

**FIGS. 16J-K** show implants in the sagittal plane with the bearing surface of the two components **1601** and **1602** having variable radii in the sagittal plane, and the second surface **1607** of the bearing component **1601** facing the second component **1603** (e.g., the femoral condyle) having also variable radii, in accordance with various embodiments of the invention. In **FIG. 16J****,** the smaller radii of the top surface facing the second component **1603** are seen centrally to posteriorly. In **FIG. 16K****,** the smaller radii of the top surface facing the second component **1603** are seen anteriorly.

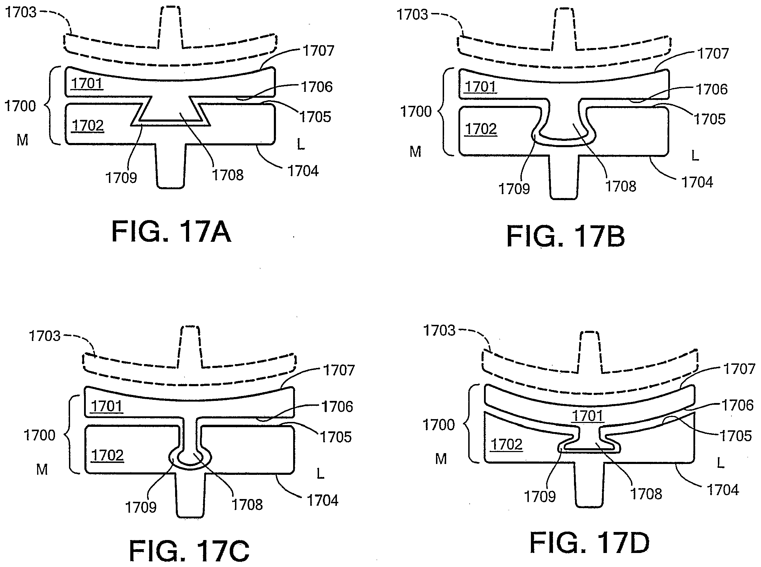

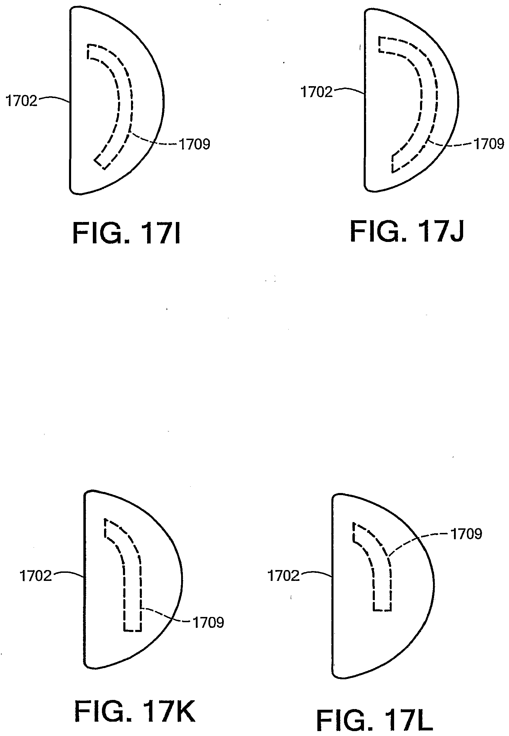

**Figs. 17a****-d** show joint implants **1700** wherein the bearing component **1701** is slideably engaged with the first component **1702,** in accordance with various embodiments of the invention. The first surface **1706** of the bearing component **1701** includes an anchor **1708** (also referred to as an extender) that runs in a recessed slot **1709** in the first component **1702,** thereby reducing the risk of dislocation of the top component relative to the bottom component.

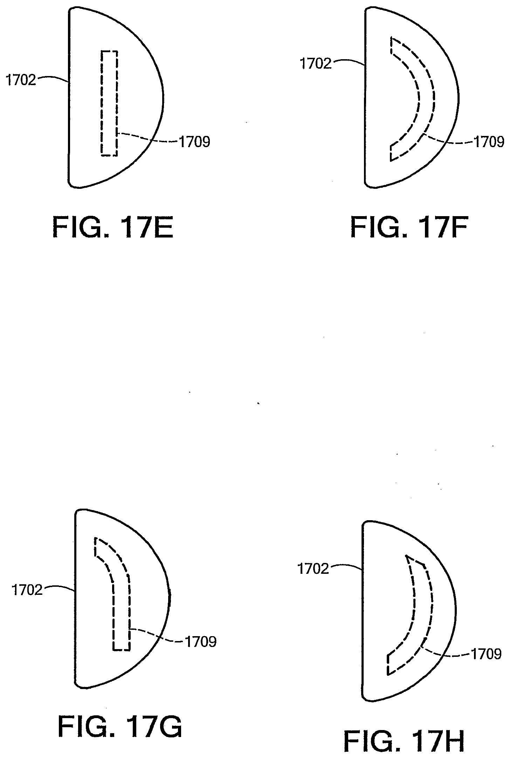

**FIGS. 17E-J** show exemplary locations and configurations of the recessed slot **1709** of the first component **1702,** in accordance with various embodiments of the invention. The shape of the recessed slot **1709** determines the direction in which the bearing component **1701** moves during gait or other knee activities. In **FIG. 17E****,** only straight AP motion is possible. In **FIG. 17F****,** the bearing component **1701** can move along a constant radius relative to the first component **1702.** In **FIG. 17G****,** the bearing component **1701** can move in anteroposterior direction; far anteriorly some rotation of the bearing component **1701** is enabled. In **FIG. 17H****,** rotation of the bearing component **1701** will occur posteriorly, the recessed slot **1709** curved only posteriorly.

**FIGS. 17I-J** show embodiments where the radius of the recessed slot **1709** is variable in the axial plane thereby allowing not only AP movement, but also gradual, constant rotation of the bearing component **1701** with knee flexion and extension and gait.

**FIG. 17K** is an example of a joint device **1700** with a recessed slot **1704** allowing extensive AP motion and anteriorly also some rotation of the bearing component **1701.** In Fig. **17L****,** the AP motion is restricted by shortening the length of the recessed slot **1704** in that dimension.

In further embodiments, the recessed slot **1704** may have a changing slope, further guiding the motion of the bearing component **1701.**

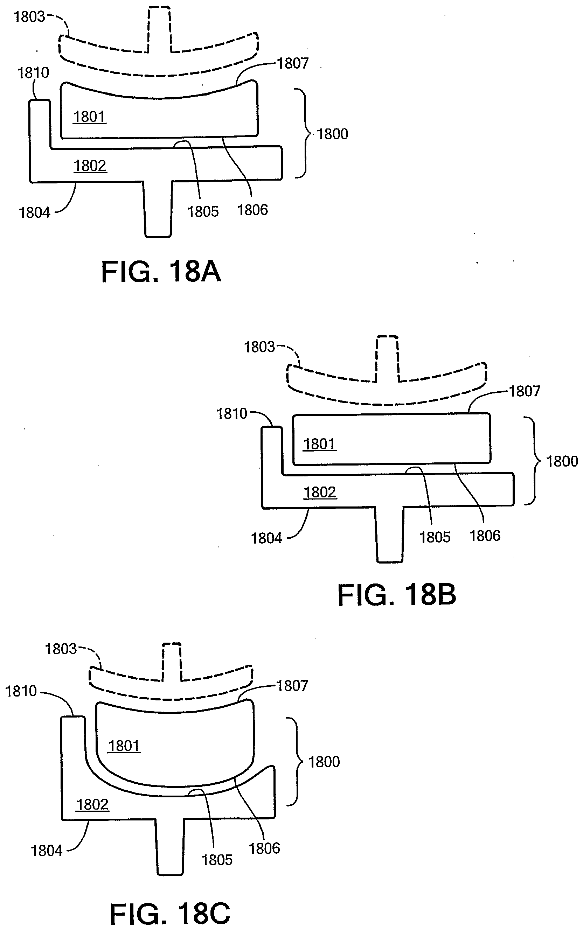

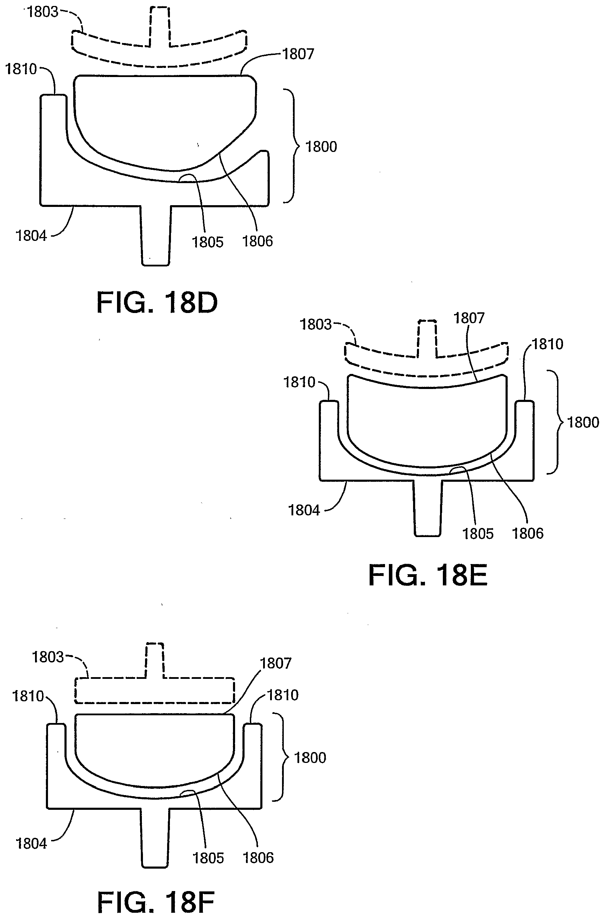

**FIGS. 18A-F** show a mobile bearing joint implant **1800** having a stop **1810** restricting motion of the bearing component **1801** in one or more dimensions, in accordance with one embodiment of the invention. There may be, without limitation, one stop **(****FIGS. 18A-D**) or two stops (**FIGS. 18E-F**).

**FIG. 18A** shows a joint implant **1800** with a flat bearing surface in one or more dimensions between the bearing component **1801** and the first component **1802,** a curvature on the second surface **1807** of the bearing component **1801** surface and a stop **1810** on the left restricting movement of the bearing component **1801** in this direction, in accordance with one embodiment of the invention. The stop **1810** is typically be located near the intercondylar notch, restricting movement of the bearing component **1801** in this direction. The stop, may be, without limitation, straight or curved. The stop may be sloped, allowing for increasing resistance, as opposed to an abrupt stop.

**FIG. 18B** shows a joint implant **1800** with a flat bearing surface in one or more dimensions between the bearing component **1801** and the first component **1802,** a flat second surface **1807** of the bearing component **1801** surface facing the second component **1803** and a stop **1810** on the left restricting movement of the bearing component **1801** in this direction, in accordance with one embodiment of the invention.

**Fig. 18C** shows a joint implant **1800** with a curved bearing surface in one or more dimensions between the bearing component **1801** and the first component **1802,** a curvature on the second surface **1807** of the bearing component **1801** surface and a stop **1810** on the left restricting movement of the bearing component **1801** in this direction, in accordance with one embodiment of the invention. The radius of the bearing surface between the bearing component **1801** and the first component **1802** may be substantially constant.

**FIG. 18D** shows a joint device **1800** with a curved bearing surface in one or more dimensions between the bearing component **1801** and the first component **1802,** a flat second surface **1807** of the bearing component **1801** surface in one or more dimensions and a stop **1810** on the left restricting movement of the bearing component **1801** in this direction, in accordance with one embodiment of the invention. The radius of the bearing surface between the bearing component **1801** and the first component **1802** is variable. In this example, the radius of the external surface **1805** of the first component **1802** facing the bearing component **1801** is not only variable but also differs in some areas from the radius of first surface **1806** of the bearing component **1801** facing the first component **1802.**

**FIGS. 18E-F** show joint implants **1800** with two stops **1810,** e.g. one medial and one lateral or one anterior and one posterior, in accordance with various embodiments of the invention. In **Fig. 18E****,** the bearing surface of the bearing and first component **1801** and **1802** is curved with a constant radius and the second surface **1807** of the bearing component **1801** facing, in a knee implant for example, the femur is curved with a substantially constant radius. In **Fig. 18F****,** the bearing surface of the bearing and first component **1801** and **1802** is curved with a constant radius and the second surface **1807** of the bearing component **1801** facing, for example in a knee implant, the femur is flat in one or more dimensions.

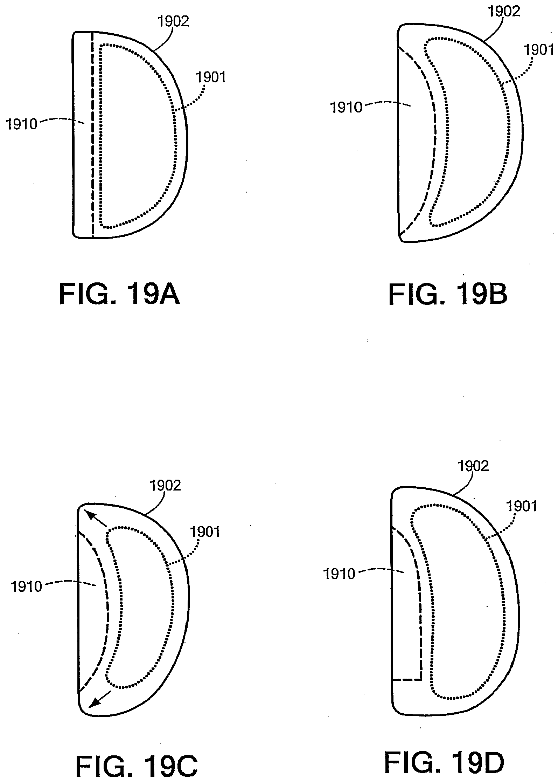

**FIGS. 19A-E** show various embodiments related to the stop **1910.** The stop 1910 may be straight, for example oriented in anteroposterior direction **(****Fig. 19A**). The stop **1910** may be curved with constant radius (**FIG. 19B**). The bearing component (stippled) **1901** may be large relative to the first component **1902,** for example covering 85% of the first component **1902,** thereby limiting the amount of anteroposterior movement and rotation (**FIG. 19B**). The bearing component (stippled) **1901** may be small relative to the first component **1902,** for example covering only 70% of the first component **1902,** thereby allowing for more anteroposterior movement and rotation (**FIG. 19C**).

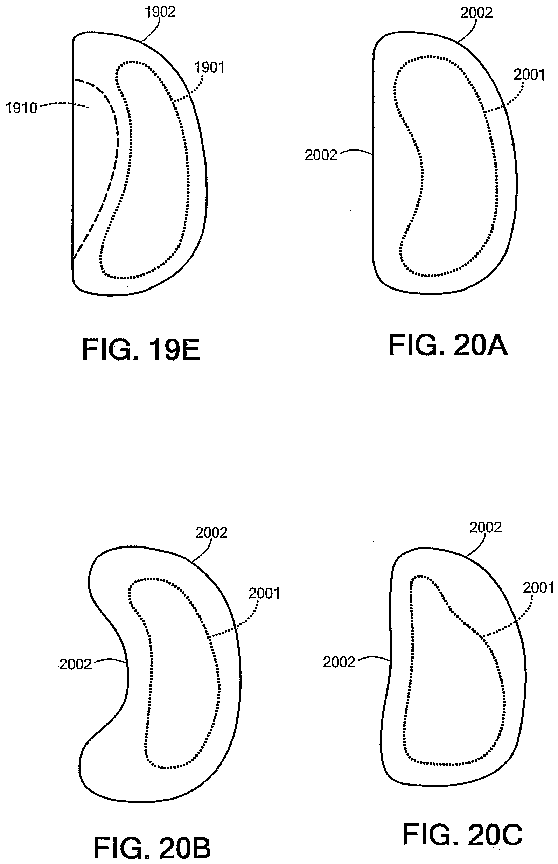

The stop **1910** may be curved with variable radius and it can contain straight portions (**FIG. 19D**). The stop **1910** may be curved with variable radius (**FIG. 19E**), for example with a substantially matching bearing component **1901** (**FIG. 19E**). With this design, the stop **1910** can influence and guide the direction of the bearing component relative to the first component. Preferably, this guidance may be used to achieve near physiologic motion.

**FIGS. 20A-C** demonstrate top (stippled line) and bottom (solid line) bearing and first components **2001** and **2002,** respectively, with different shapes, in accordance with various embodiments of the invention. The bearing component **2001** may substantially be the same or substantially differ from the shape of the first component **2002.** Both bearing and first component **2001** and **2002** may have, without limitation, one or more straight, convex or concave portions.

The various joint implants described herein may be used, without limitation, in conjunction with knee implants, including a unicompartmental arthroplasty, medial or lateral; a bicompartmental arthroplasty that covers portions or all of one femoral condyle, medial or lateral, and the trochlea, and a total knee arthroplasty system. In a total knee arthroplasty system, the intercondylar region can be preserved by using a medial and a lateral tibial device in combination. Both devices may be a fixed, non-mobile bearing, both can be a mobile bearing, or one can be a fixed, non-mobile bearing, while the other is a mobile bearing. As described above, the joint implants described herein may also be implemented for the hip, ankle, shoulder, elbow, wrist, and hand.

In various embodiments, the joint implant may have one or more mobile bearings.

The various components used for the mobile bearing joint implant may be composed of metal, plastic, ceramic or any other material know in the art. Different components may be composed of different materials, e.g. one metal and one plastic. Alternatively, only the same material may be used for the bearing surfaces, e.g. ceramic. The bearing surfaces of each component may vary in material composition e.g. ceramic on the side facing the femoral condyle and metal on the undersurface.

As described herein, repair systems of various sizes, curvatures and thicknesses can be obtained. These repair systems can be catalogued and stored to create a library of systems from which an appropriate system for an individual patient can then be selected. In other words, a defect, or an articular surface, is assessed in a particular subject and a pre-existing repair system having a suitable shape and size is selected from the library for further manipulation (*e.g.,* shaping) and implantation.

### IV. MANUFACTURING

### A. SHAPING

In certain instances shaping of the repair material will be required before or after formation (*e.g.,* growth to desired thickness), for example where the thickness of the required cartilage material is not uniform (*e.g.,* where different sections of the cartilage replacement or regenerating material require different thicknesses).

The replacement material can be shaped by any suitable technique including, but not limited to, casting techniques, mechanical abrasion, laser abrasion or ablation, radiofrequency treatment, cryoablation, variations in exposure time and concentration of nutrients, enzymes or growth factors and any other means suitable for influencing or changing cartilage thickness. See, *e.g.,* WO 00/15153 to Mansmann published March 23, 2000; If enzymatic digestion is used, certain sections of the cartilage replacement or regenerating material can be exposed to higher doses of the enzyme or can be exposed longer as a means of achieving different thicknesses and curvatures of the cartilage replacement or regenerating material in different sections of said material.

The material can be shaped manually and/or automatically, for example using a device into which a pre-selected thickness and/or curvature has been input and then programming the device using the input information to achieve the desired shape.

In addition to, or instead of, shaping the cartilage repair material, the site of implantation (*e.g.,* bone surface, any cartilage material remaining, etc.) can also be shaped by any suitable technique in order to enhance integration of the repair material.

### B. SIZING

The articular repair system can be formed or selected so that it will achieve a near anatomic fit or match with the surrounding or adjacent cartilage, subchondral bone, menisci and/or other tissue. The shape of the repair system can be based on the analysis of an electronic image (*e.g.* MRI, CT, digital tomosynthesis, optical coherence tomography or the like). If the articular repair system is intended to replace an area of diseased cartilage or lost cartilage, the near anatomic fit can be achieved using a method that provides a virtual reconstruction of the shape of healthy cartilage in an electronic image.

In one embodiment of the invention, a near normal cartilage surface at the position of the cartilage defect can be reconstructed by interpolating the healthy cartilage surface across the cartilage defect or area of diseased cartilage. This can, for example, be achieved by describing the healthy cartilage by means of a parametric surface (*e.g.* a B-spline surface), for which the control points are placed such that the parametric surface follows the contour of the healthy cartilage and bridges the cartilage defect or area of diseased cartilage. The continuity properties of the parametric surface will provide a smooth integration of the part that bridges the cartilage defect or area of diseased cartilage with the contour of the surrounding healthy cartilage. The part of the parametric surface over the area of the cartilage defect or area of diseased cartilage can be used to determine the shape or part of the shape of the articular repair system to match with the surrounding cartilage.

In another embodiment, a near normal cartilage surface at the position of the cartilage defect or area of diseased cartilage can be reconstructed using morphological image processing. In a first step, the cartilage can be extracted from the electronic image using manual, semi-automated and/or automated segmentation techniques (*e.g.,* manual tracing, region growing, live wire, model-based segmentation), resulting in a binary image. Defects in the cartilage appear as indentations that can be filled with a morphological closing operation performed in 2-D or 3-D with an appropriately selected structuring element. The closing operation is typically defined as a dilation followed by an erosion. A dilation operator sets the current pixel in the output image to 1 if at least one pixel of the structuring element lies inside a region in the source image. An erosion operator sets the current pixel in the output image to 1 if the whole structuring element lies inside a region in the source image. The filling of the cartilage defect or area of diseased cartilage creates a new surface over the area of the cartilage defect or area of diseased cartilage that can be used to determine the shape or part of the shape of the articular repair system to match with the surrounding cartilage or subchondral bone.

As described above, the articular repair system can be formed or selected from a library or database of systems of various sizes, curvatures and thicknesses so that it will achieve a near anatomic fit or match with the surrounding or adjacent cartilage and/or subchondral bone. These systems can be pre-made or made to order for an individual patient. In order to control the fit or match of the articular repair system with the surrounding or adjacent cartilage or subchondral bone or menisci and other tissues preoperatively, a software program can be used that projects the articular repair system over the anatomic position where it will be implanted. Suitable software is commercially available and/or readily modified or designed by a skilled programmer.

In yet another embodiment, the articular surface repair system can be projected over the implantation site using one or more 3-D images. The cartilage and/or subchondral bone and other anatomic structures are extracted from a 3-D electronic image such as an MRI or a CT using manual, semi-automated and/or automated segmentation techniques. A 3-D representation of the cartilage and/or subchondral bone and other anatomic structures as well as the articular repair system is generated, for example using a polygon or NURBS surface or other parametric surface representation. For a description of various parametric surface representations see, for example Foley, J.D. et al., Computer Graphics: Principles and Practice in C; Addison-Wesley, 2nd edition, 1995).

The 3-D representations of the cartilage and/or subchondral bone and other anatomic structures and the articular repair system can be merged into a common coordinate system. The articular repair system can then be placed at the desired implantation site. The representations of the cartilage, subchondral bone, menisci and other anatomic structures and the articular repair system are rendered into a 3-D image, for example application programming interfaces (APIs) OpenGL® (standard library of advanced 3-D graphics functions developed by SGI, Inc.; available as part of the drivers for PC-based video cards, for example from www.nvidia.com for NVIDIA video cards or www.3dlabs.com for 3Dlabs products, or as part of the system software for Unix workstations) or DirectX® (multimedia API for Microsoft Windows® based PC systems; available from www.microsoft.com). The 3-D image can be rendered showing the cartilage, subchondral bone, menisci or other anatomic objects, and the articular repair system from varying angles, *e.g.* by rotating or moving them interactively or non-interactively, in real-time or non-real-time.

The software can be designed so that the articular repair system, including surgical tools and instruments with the best fit relative to the cartilage and/or subchondral bone is automatically selected, for example using some of the techniques described above. Alternatively, the operator can select an articular repair system, including surgical tools and instruments and project it or drag it onto the implantation site using suitable tools and techniques. The operator can move and rotate the articular repair systems in three dimensions relative to the implantation site and can perform a visual inspection of the fit between the articular repair system and the implantation site. The visual inspection can be computer assisted. The procedure can be repeated until a satisfactory fit has been achieved. The procedure can be performed manually by the operator; or it can be computer-assisted in whole or part. For example, the software can select a first trial implant that the operator can test. The operator can evaluate the fit. The software can be designed and used to highlight areas of poor alignment between the implant and the surrounding cartilage or subchondral bone or menisci or other tissues. Based on this information, the software or the operator can then select another implant and test its alignment. One of skill in the art will readily be able to select, modify and/or create suitable computer programs for the purposes described herein.

In another embodiment, the implantation site can be visualized using one or more cross-sectional 2-D images. Typically, a series of 2-D cross-sectional images will be used. The 2-D images can be generated with imaging tests such as CT, MRI, digital tomosynthesis, ultrasound, or optical coherence tomography using methods and tools known to those of skill in the art. The articular repair system can then be superimposed onto one or more of these 2-D images. The 2-D cross-sectional images can be reconstructed in other planes, e.g. from sagittal to coronal, etc. Isotropic data sets (*e.g.,* data sets where the slice thickness is the same or nearly the same as the in-plane resolution) or near isotropic data sets can also be used. Multiple planes can be displayed simultaneously, for example using a split screen display. The operator can also scroll through the 2-D images in any desired orientation in real time or near real time; the operator can rotate the imaged tissue volume while doing this. The articular repair system can be displayed in cross-section utilizing different display planes, *e.g.* sagittal, coronal or axial, typically matching those of the 2-D images demonstrating the cartilage, subchondral bone, menisci or other tissue. Alternatively, a three-dimensional display can be used for the articular repair system. The 2-D electronic image and the 2-D or 3-D representation of the articular repair system can be merged into a common coordinate system. The articular repair system can then be placed at the desired implantation site. The series of 2-D cross-sections of the anatomic structures, the implantation site and the articular repair system can be displayed interactively (*e.g.* the operator can scroll through a series of slices) or non-interactively (*e.g.* as an animation that moves through the series of slices), in real-time or non-real-time.

### C. RAPID PROTOTYPING

Rapid prototyping is a technique for fabricating a three-dimensional object from a computer model of the object. A special printer is used to fabricate the prototype from a plurality of two-dimensional layers. Computer software sections the representations of the object into a plurality of distinct two-dimensional layers and then a three-dimensional printer fabricates a layer of material for each layer sectioned by the software. Together the various fabricated layers form the desired prototype. More information about rapid prototyping techniques is available in US Patent Publication No 2002/0079601A1 to Russell et al., published June 27, 2002. An advantage to using rapid prototyping is that it enables the use of free form fabrication techniques that use toxic or potent compounds safely. These compounds can be safely incorporated in an excipient envelope, which reduces worker exposure

A powder piston and build bed are provided. Powder includes any material (metal, plastic, etc.) that can be made into a powder or bonded with a liquid. The power is rolled from a feeder source with a spreader onto a surface of a bed. The thickness of the layer is controlled by the computer. The print head then deposits a binder fluid onto the powder layer at a location where it is desired that the powder bind. Powder is again rolled into the build bed and the process is repeated, with the binding fluid deposition being controlled at each layer to correspond to the three-dimensional location of the device formation. For a further discussion of this process see, for example, US Patent Publication No 2003/017365A1 to Monkhouse et al. published September 18, 2003.

The rapid prototyping can use the two dimensional images obtained, as described above in Section I, to determine each of the two-dimensional shapes for each of the layers of the prototyping machine. In this scenario, each two dimensional image slice would correspond to a two dimensional prototype slide. Alternatively, the three-dimensional shape of the defect can be determined, as described above, and then broken down into two dimensional slices for the rapid prototyping process. The advantage of using the three-dimensional model is that the two-dimensional slices used for the rapid prototyping machine can be along the same plane as the two-dimensional images taken or along a different plane altogether.

Rapid prototyping can be combined or used in conjunction with casting techniques. For example, a shell or container with inner dimensions corresponding to an articular repair system can be made using rapid prototyping. Plastic or wax-like materials are typically used for this purpose. The inside of the container can subsequently be coated, for example with a ceramic, for subsequent casting. Using this process, personalized casts can be generated.

Rapid prototyping can be used for producing articular repair systems. Rapid prototyping can be performed at a manufacturing facility. Alternatively, it may be performed in the operating room after an intraoperative measurement has been performed.

### V. SURGICAL TECHNIQUES

Prior to performing surgery on a patient, the surgeon can preoperatively make a determination of the alignment of the knee using, for example, an erect AP x-ray. In performing preoperative assessment any lateral and patella spurs that are present can be identified.

Using standard surgical techniques, the patient is anesthetized and an incision is made in order to provide access to the portion or portions of the knee joint to be repaired. A medial portal can be used initially to enable arthroscopy of the joint. Thereafter, the medial portal can be incorporated into the operative incision and/or standard lateral portals can be used.

Once an appropriate incision has been made, the exposed compartment is inspected for integrity, including the integrity of the ligament structures. If necessary, portions of the meniscus can be removed as well as any spurs or osteophytes that were identified in the AP x-ray or that may be present within the joint. In order to facilitate removal of osteophytes, the surgeon may flex the knee to gain exposure to additional medial and medial-posterior osteophytes. Additionally, osteophytes can be removed from the patella during this process. If necessary, the medial and/or lateral meniscus can also be removed at this point, if desired, along with the rim of the meniscus.

As would be appreciated by those of skill in the art, evaluation of the medial cruciate ligament may be required to facilitate tibial osteophyte removal.

Once the joint surfaces have been prepared, the desired implants can be inserted into the joint.

### A. Tibial Plateau

To insert the device ***200*** of **FIG. 2** into the medial compartment, perform a mini-incision arthrotomy medial to the patella tendon. Once the incision is made, expose the medial condyle and prepare a medial sleeve to about 1 cm below the joint line using a suitable knife and curved osteotome. After preparing the medial sleeve, place a Z-retractor around the medial tibial plateau and remove anterior portions of the meniscus and the osteophytes along the tibia and femur. At this point, the knee should be flexed to about 60° or more. Remove the Z-retractor and place the implant against the most distal aspect of the femur and over the tibial plateau edge. Push the implant straight back. In some instances, application of valgus stress may ease insertion of the implant.

To insert the device of **FIG. 2** into the lateral compartment, perform a mini-incision arthrotomy lateral to the patella tendon. Once the incision is made, expose the lateral condyle and prepare a lateral sleeve to about 1 cm below the joint line using a suitable knife and curved osteotome. After preparing the lateral sleeve, place a Z-retractor around the lateral tibial plateau and remove anterior portions of the meniscus and the osteophytes along the tibia and femur. Remove the Z-retractor and place the implant against the distal aspect of the femur and over the tibial plateau edge. Hold the implant at a 45° angle and rotate the implant against the lateral condyle using a lateral to medial push toward the lateral spine. In some instances, application of varus stress may ease insertion of the implant.

Once any implant shown in **FIG. 2** is implanted, the device should be positioned within 0 to 2mm of the AP boundaries of the tibial plateau and superimposed over the boundary. Verification of the range of motion should then be performed to confirm that there is minimal translation of the implant. Once positioning is confirmed, closure of the wound is performed using techniques known in the art.

As will be appreciated by those of skill in the art, additional treatment of the surface of the tibial plateau may be desirable depending on the configuration of the implant ***200.*** For example, one or more channels or grooves may be formed on the surface of the tibial plateau to accommodate anchoring mechanisms such as the keel ***212*** shown in **FIG. 2K** or the translational movement cross-members ***222, 221*** shown in **FIGS. 2M-N****.**

### B. Condylar Repair Systems

To insert the device ***300*** shown in **FIG. 3****,** depending on the condyle to be repaired either an antero-medial or antero-lateral skin incisions is made which begins approximately 1 cm proximal to the superior border of the patella. The incision typically can range from, for example, 6-10 cm along the edge of the patella. As will be appreciated by those of skill in the art, a longer incision may be required under some circumstances.

It may be required to excise excess deep synovium to improve access to the joint. Additionally, all or part of the fat pad may also be excused and to enable inspection of the opposite joint compartment.

Typically, osteophytes are removed from the entire medial and/or lateral edge of the femur and the tibia as well as any osteophytes on the edge of the patella that might be significant.

Although it is possible, typically the devices ***300*** do not require resection of the distal femur prior to implanting the device. However, if desired, bone cuts can be performed to provide a surface for the implant.

At this juncture, the patient's leg is placed in 90° flexion position. I guide can then be placed on the condyle which covers the distal femoral cartilage. The guide enables the surgeon to determine placement of apertures that enable the implant ***300*** to be accurately placed on the condyle. With the guide in place, holes are drilled into the condyle to create apertures from 1-3mm in depth. Once the apertures have been created, the guide is removed and the implant ***300*** is installed on the surface of the condyle. Cement can be used to facilitate adherence of the implant ***300*** to the condyle.

Where more than one condyle is to be repaired, e.g., using two implants ***300*** of **FIG. 3****,** or the implant ***400*** of **FIG. 4****,** or where a condyle and a portion of the patellar surface is to be repaired, e.g., using the implant ***500*** of **FIG. 5****,** the surgical technique described herein would be modified to, for example, provide a greater incision for accessing the joint, provide additional apertures for receiving the pegs of the implant, etc.

### C. Patellar Repair System

To insert the device shown in **FIG. 7****,** it may be appropriate to use the incisions made laterally or medially to the patella tendon and described above with respect to **FIG. 2****.** First the patella is everted laterally and the fat pad and synovium are bent back from around the periphery of the patella. If desired, osteophytes can be removed. Prior to resurfacing the natural patella ***620,*** the knee should be manually taken through several range of motion maneuvers to determine whether subluxation is present. If subluxation is present, then it may be necessary to medialize the implant ***600.*** The natural patella can then be cut in a planar, or flat, manner such that a flat surface is presented to the implant. The geometric center of the patella ***620*** is then typically aligned with the geometric center of the implant ***600.*** In order to anchor the implant ***600*** to the patella ***620,*** one or more holes or apertures ***612*** can be created in the patellar surface to accept the pegs ***610*** of the implant ***600.***

### VI. KITS

One ore more of the implants described above can be combined together in a kit such that the surgeon can select one or more implants to be used during surgery.

The foregoing description of embodiments of the present invention has been provided for the purposes of illustration and description. It is not intended to be exhaustive or to limit the invention to the precise forms disclosed. Many modifications and variations will be apparent to the practitioner skilled in the art. The embodiments were chosen and described in order to best explain the principles of the invention and its practical application, thereby enabling others skilled in the art to understand the invention and the various embodiments and with various modifications that are suited to the particular use contemplated.

### DISCLOSED ITEMS

1. A mobile bearing implant, the implant comprising:
   a first component including an bone facing surface and an external surface, the bone facing surface for engaging one of bone or cartilage, the external surface including a slot;
   a bearing component having a first surface and a second surface; the first surface for slidingly engaging the external surface of the first component, the second surface for engaging at least one of a second component, bone surface, and cartilage, wherein the first surface of the bearing component includes an anchor, the anchor slidingly engaging the slot so as to direct movement of the bearing component along the external surface of the first component.
2. The implant according to item 1, wherein the bone facing surface of the first component engages a tibial articular surface, and wherein the second surface of the bearing component engages a femoral implant component.
3. The implant according to item 1, wherein the slot is curved.
4. The implant according to item 3, wherein the slot includes a plurality of curvatures with varying radii.
5. The implant according to item 1, wherein the slot is sloped.
6. The implant according to item 1, wherein the first component includes at least one stop for limiting motion of the bearing component, the stop including a curved surface for contacting the bearing component.
7. A mobile bearing implant, the implant comprising:
   a first component including a bone facing surface and an external surface, the bone facing surface for engaging one of a substantially uncut articular cartilage surface and a substantially uncut subchondral bone surface, the bone facing surface substantially matching the one of the articular cartilage surface and the subchondral bone surface,
   a bearing component having a first surface and a second surface; the first surface for slidingly engaging the external surface of the first component, the second surface for engaging at least one of a second component, bone, and cartilage.
8. The implant according to item 7, wherein the bone facing surface of the first component substantially matches one of a substantially uncut articular cartilage surface of a tibia and a substantially uncut subchondral bone surface of a tibia, and wherein the second surface of the bearing component engages a femoral implant component.
9. The implant according to item 7, wherein the external surface of the first component is curved.
10. The implant according to item 9, wherein the external surface includes a plurality of curved surfaces with varying radii.
11. The implant according to item 10, wherein the external surface is flat along at least one axis.
12. The implant according to item 7, wherein the external surface of the first component includes a slot, and wherein the first surface of the bearing component includes an anchor, the anchor slidingly engaging the slot so as to direct movement of the bearing component along the external surface of the first component.
13. The implant according to item 12, wherein the slot is curved.
14. The implant according to item 13, wherein the slot includes a plurality of curvatures with varying radii.
15. The implant according to item 12, wherein the slot is sloped.
16. A mobile bearing implant, the implant comprising:
   a first component including a bone facing surface and an external surface; the bone facing surface for engaging at least one of bone and cartilage;
   a bearing component having a first surface and a second surface; the first surface for slidingly engaging the external surface of the first component, the second surface for engaging at least one of a second component, bone, and cartilage, wherein the external surface of the first component includes at least one of a concavity and a convexity.
17. The implant according to item 16, wherein the external surface of the first component includes a plurality of curved surfaces with varying radii.
18. The implant according to item 16, wherein the bone facing surface of the first component engages a tibial articular surface, and wherein the second surface of the bearing component engages a femoral implant component.
19. The implant according to item 16, wherein the external surface is flat along at least one axis.
20. The implant according to item 16, wherein the external surface of the first component includes a slot, and wherein the first surface of the bearing component includes an anchor, the anchor slidingly engaging the slot so as to direct movement of the bearing component along the external surface of the first component.
21. The implant according to item 20, wherein the slot is curved.
22. The implant according to item 21, wherein the slot includes a plurality of curvatures with varying radii.
23. The implant according to item 20, wherein the slot is sloped.
24. The implant according to item 16, wherein the first component includes at least one stop for limiting motion of the bearing component, the stop including a curved surface for contacting the bearing component.
25. A mobile bearing implant, the implant comprising:
   a first component including an bone facing surface and an external surface, the bone facing surface for engaging one of bone or cartilage, the external surface including a slot;
   a bearing component having a first surface and a second surface; the first surface for slidingly engaging the external surface of the first component, the second surface for engaging at least one of a second component, bone surface, and cartilage, wherein the first surface of the bearing component includes an anchor, the anchor slidingly engaging the slot so as to direct movement of the bearing component along the external surface of the first component.
26. The implant according to item 25, wherein the bone facing surface of the first component engages a tibial articular surface, and wherein the second surface of the bearing component engages a femoral implant component.
27. The implant according to item 25, wherein the slot is curved.
28. The implant according to item 27, wherein the slot includes a plurality of curvatures with varying radii.
29. The implant according to item 25, wherein the slot is sloped.
30. A mobile bearing implant, the implant comprising:
   a first component including a bone facing surface and an external surface; the bone facing surface for engaging one of bone and cartilage;
   a bearing component having a first surface and a second surface; the first surface for slidingly engaging the external surface of the first component, the second surface for engaging at least one of a second component, bone, and cartilage, wherein at least one of the first surface and the second surface includes a curved surface in one dimension, the curved surface having a plurality of radii.
31. A mobile bearing implant, the implant comprising:
   a first component including a bone facing surface and an external surface; the bone facing surface for engaging one of bone and cartilage;
   a bearing component having a first surface and a second surface; the first surface for slidingly engaging the external surface of the first component, the second surface for engaging at least one of a second component, bone, and cartilage, wherein the first component has an outer perimeter of varying radii.
32. The mobile bearing according to item 31, wherein the outer perimeter of the first component is kidney shaped.
33. The mobile bearing according to item 31, wherein the outer perimeter of the first component is asymmetric.
34. The mobile bearing according to item 31, wherein the first component has a larger outer perimeter than the bearing component.
35. The mobile bearing according to item 31, wherein the first component has a smaller outer perimeter than the bearing component.

## Claims

1. A patient -specific mobile bearing implant for repairing a patient's joint, the implant comprising:
a first component for engaging a surface of a tibia of the patient's joint;
a second component for covering at least a portion of a femoral condyle of the patient's joint and having varying radii in at least one of an anteroposterior and mediolateral direction, wherein the varying radii provide a patient-specific curvature that substantially matches an adjacent portion of the patient's joint,
a bearing component having a first surface and a second surface; the first surface for slidingly engaging a external surface of the first component, the second surface for articulating with the second component and having variying radii that substantially matching the radii of the second component.

2. The implant according to claim 1, wherein the external surface of the first component includes a slot, and wherein the first surface of the bearing component includes an anchor, the anchor slidingly engaging the slot so as to direct movement of the bearing component along the external surface of the first component.

3. The implant according to claim 1, wherein the first component includes at least one stop for limiting motion of the bearing component, the stop including a curved surface for contacting the bearing component.

4. The implant according to claim 2, wherein the slot is curved or sloped or includes a plurality of curvatures with varying radii.

5. A patient-specific mobile bearing implant for repairing a patient's joint, the implant comprising:
a first component including a bone facing surface and an external surface; the bone facing surface for engaging a tibia of the patient's joint,
a second component for covering at least a portion of a condyle of the patient's joint and having varying radii in at least one of an anteroposterior and mediolateral direction, wherein the varying radii provide a patient-specific curvature that substantially matches an adjacent portion of the patient's joint,
a bearing component having a first surface and a second surface; the first surface for slidingly engaging the external surface of the first component, the second surface for articulating with the second component, wherein the second component is thereby movable relative to the first component in an anteroposterior direction.

6. The implant according to claim 5, wherein the external surface of the first component includes a plurality of curved surfaces with varying radii.

7. The implant according to claim 5, wherein the bone facing surface of the first component engages a tibial articular surface, and wherein the second surface of the bearing component engages a femoral implant component.

8. The implant according to claim 5, wherein the external surface of the first component includes a slot, and wherein the first surface of the bearing component includes an anchor, the anchor slidingly engaging the slot so as to direct movement of the bearing component along the external surface of the first component.

9. The implant according to claim 8, wherein the slot is sloped or curved or includes a plurality of curvatures with varying radii.

10. The implant according to claim 5, wherein the first component includes at least one stop for limiting motion of the bearing component, the stop including a curved surface for contacting the bearing component.

11. A patient-specific mobile bearing implant for repairing a patient's joint, the implant comprising:
a first component for engaging a tibia of the patient's joint and including an bone facing surface and an external surface, wherein the external surface is curved in a sagittal plane;
a second component for covering at least a portion of a condyle of the patient's joint and having varying radii in at least one of an anteroposterior and mediolateral direction, wherein the varying radii provide a patient-specific curvature that substantially matches an adjacent portion of the patient's joint,
a bearing component having a first surface and a second surface; the first surface having a curvature in the sagittal plane for slidingly engaging the external surface of the first component, the second surface for articulating with the second component.

12. The implant according to claim 11, wherein the bone facing surface of the first component engages a tibial articular surface, and wherein the second surface of the bearing component engages a femoral implant component.

13. A patient-specific mobile bearing implant for repairing a patient's joint, the implant comprising:
a first component including a bone facing surface and an external surface; the bone facing surface for engaging a tibia of the patient's joint;
a second component for covering at least a portion of a femoral condyle of the patient's joint and having varying radii in an anteroposterior direction, wherein the varying radii provide a patient-specific curvature that substantially matches an adjacent portion of the patient's joint;
a bearing component having a first surface and a second surface; the first surface for slidingly engaging the external surface of the first component, the second surface for engaging second component, bone, and cartilage, wherein at least one of the first surface and the second surface includes a curved surface in one dimension, the curved surface having a plurality of radii.

14. A patient-specific mobile bearing implant for repairing a patient's joint, the implant comprising:
a first component including a bone facing surface and an external surface; the bone facing surface for engaging a surface of the patient's joint;
a second component for covering at least a portion of a surface of the patient's joint and having varying radii in at least one direction, wherein the varying radii provide a patient-specific curvature that substantially matches an adjacent portion of the patient's joint,
a bearing component having a first surface and a second surface; the first surface for slidingly engaging the external surface of the first component, the second surface for articulating with the second component, wherein the second component is thereby movable relative to the first component.

15. The mobile bearing implant according to claim 14, wherein the outer perimeter of the first component is asymmetric.
